(19) Europäisches Patentamt
European Patent Office
Office européen des brevets

(11) EP 0 740 659 B1

(12) **EUROPÄISCHE PATENTSCHRIFT**

(45) Veröffentlichungstag und Bekanntmachung des Hinweises auf die Patenterteilung:
**05.04.2000 Patentblatt 2000/14**

(21) Anmeldenummer: **95905117.8**

(22) Anmeldetag: **04.01.1995**

(51) Int. Cl.$^7$: **C07D 263/10**, A01N 43/76, C07D 263/14, C07F 7/18

(86) Internationale Anmeldenummer:
**PCT/EP95/00020**

(87) Internationale Veröffentlichungsnummer:
**WO 95/19348 (20.07.1995 Gazette 1995/31)**

(54) **PHENYLTHIO-OXAZOLIN-DERIVATE**
PHENYLTHIO-OXAZOLINE DERIVATIVES
DERIVES DE PHENYLTHIO-OXAZOLINE

(84) Benannte Vertragsstaaten:
**BE CH DE ES FR GB IT LI NL**

(30) Priorität: **17.01.1994 DE 4401101**

(43) Veröffentlichungstag der Anmeldung:
**06.11.1996 Patentblatt 1996/45**

(73) Patentinhaber: **BAYER AG**
**51368 Leverkusen (DE)**

(72) Erfinder:
- **KRÄMER, Wolfgang**
  **D-51399 Burscheid (DE)**
- **WACHENDORFF-NEUMANN, Ulrike**
  **D-40789 Monheim (DE)**
- **ERDELEN, Christoph**
  **D-42799 Leichlingen (DE)**
- **TURBERG, Andreas**
  **D-40699 Erkrath (DE)**
- **MENCKE, Norbert**
  **D-51381 Leverkusen (DE)**

(56) Entgegenhaltungen:
**EP-A- 0 168 813** **EP-A- 0 345 775**
**EP-A- 0 553 623**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 740 659 B1

**Beschreibung**

Die Erfindung betrifft neue Phenylthio-oxazolin-Derivate, mehrere Verfahren zu ihrer Herstellung und ihre Verwendung zur Bekämpfung von tierischen Schädlingen.

Es ist bereits bekannt, daß bestimmte Oxazolin-Derivate insektizide und akarizide Eigenschaften aufweisen (vgl. z.B. EP-A 0 533 623). Die Wirksamkeit dieser vorbekannten Verbindungen ist jedoch, insbesondere bei niedrigen Aufwandmengen und Konzentrationen nicht in allen Anwendungsgebieten völlig zufriedenstellend.

Es wurden nun die neuen Phenylthio-oxazolin-Derivate der Formel (I) gefunden,

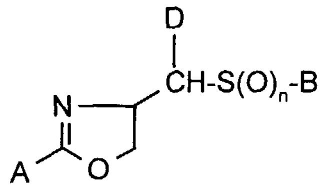

(I)

in welcher

A für Phenyl steht, das einfach bis fünffach, gleich oder verschieden substituiert ist durch
Halogen,
$C_1$-$C_6$-Alkyl,
$C_1$-$C_6$-Alkoxy,
$C_1$-$C_6$-Alkylthio,
$C_1$-$C_6$-Halogenalkyl,
$C_1$-$C_6$-Halogenalkoxy oder $C_1$-$C_6$-Halogenalkylthio;

B für Phenyl steht, das gegebenenfalls einfach bis vierfach, gleich oder verschieden substituiert ist durch
Halogen,
$C_1$-$C_{18}$-Alkyl,
Cyano,
$C_1$-$C_8$-Alkoxy-$C_1$-$C_8$-alkyl,
$C_1$-$C_8$-Halogenalkoxy,
$C_1$-$C_4$-Halogenalkyl,
$C_1$-$C_{18}$-Alkoxy, das gegebenenfalls durch weitere 1-3 Sauerstoffatome unterbrochen ist,
$C_1$-$C_{18}$-Alkylthio,
$C_1$-$C_8$-Halogenalkylthio,
3,4-Difluormethylendioxo,
3,4-Tetrafluorethylendioxo,
gegebenenfalls durch $C_1$-$C_4$-Alkyl, $C_3$-$C_6$-Cycloalkyl und/oder Halogen substituiertes Benzyliminooxymethyl,
jeweils gegebenenfalls durch $C_1$-$C_6$-Alkyl, $C_1$-$C_6$-Alkoxy, Cyclohexyl oder Phenyl substituiertes Cyclohexyl oder Cyclohexyloxy;
gegebenenfalls einfach oder zweifach, gleich oder verschieden durch Halogen, $C_1$-$C_4$-Alkyl oder $C_1$-$C_4$-Halogenalkyl substituiertes Pyridyloxy;
jeweils gegebenenfalls einfach bis dreifach, gleich oder verschieden durch $C_1$-$C_{12}$-Alkyl, Halogen, $C_1$-$C_4$-Halogenalkyl, $C_1$-$C_6$-Alkoxy, $C_1$-$C_6$-Halogenalkoxy, $C_1$-$C_6$-Alkoxy-$C_1$-$C_6$-alkyl, $C_1$-$C_6$-Alkoxy-ethylenoxy, $C_1$-$C_6$-Alkylthio und/oder $C_1$-$C_6$-Halogenalkylthio substituiertes Phenyl, Phenylcarbonyl, Benzyl, Phenoxy, Phenylthio, Benzyloxy, Benzyloxycarbonyl oder Benzylthio;
$C_1$-$C_{12}$-Alkyl-carbonyl,
$C_1$-$C_{12}$-Alkoxy-carbonyl,
$C_5$-$C_7$-Cycloalkyloxy-carbonyl, das gegebenenfalls durch $C_1$-$C_6$-Alkyl und/oder $C_1$-$C_6$-Alkoxy substituiert ist;
$C_5$-$C_7$-Cycloalkyl-$C_1$-$C_6$-alkyloxy-carbonyl, das gegebenenfalls im Cycloalkylteil durch $C_1$-$C_6$-Alkyl und/oder $C_1$-$C_6$-Alkoxy substituiert ist; und
die Gruppierung $-CO-NY^1Y^2$, wobei
$Y^1$ und $Y^2$ gleich oder verschieden sind und folgende Bedeutungen haben: Wasserstoff,
$C_1$-$C_6$-Alkyl,
$C_5$-$C_7$-Cycloalkyl, das gegebenenfalls durch $C_1$-$C_6$-Alkyl und/oder $C_1$-$C_6$-Alkoxy substituiert ist, sowie
Benzyl, das gegebenenfalls durch die o.g. Benzylsubstituenten substituiert ist,

D für Wasserstoff oder Methyl steht und

n für 0, 1 oder 2 steht.

Weiterhin wurde gefunden, daß man Verbindungen der Formel (I) erhält, wenn man

a) Aminalkohole der Formel (II)

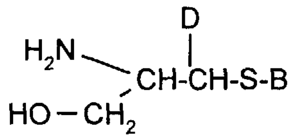

(II)

in welcher
B und D die oben angegebene Bedeutung haben,
mit einer Carbonsäure der Formel (III)

A-COOH (III)

in welcher

A die oben angegebene Bedeutung hat,

mit einem wasser-entziehenden Mittel gegebenenfalls in Gegenwart eines Verdünnungsmittels umsetzt; oder

b) Amidalkohole der Formel (IV)

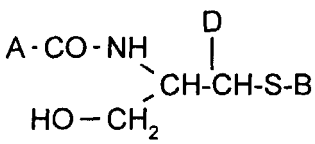

(IV)

in welcher
A, B und D die oben angegebene Bedeutung haben,
mit einem wasser-entziehenden Mittel, gegebenenfalls in Gegenwart eines Verdünnungsmittels umsetzt; oder

c) Amid-Derivate der Formel (V)

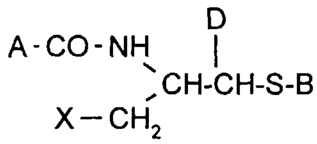

(V)

in welcher
A, B und D die oben angegebene Bedeutung haben; und

X für eine Abgangsgruppe, wie Halogen, Alkylsulfonyloxy oder Arylsulfonyloxy steht,

mit einer Base, gegebenenfalls in Gegenwart eines Verdünnungsmittels umsetzt;
oder

d) Oxazoline der Formel (VI)

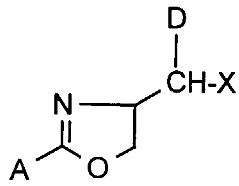

(VI)

in welcher
A, D und X die oben angegebene Bedeutung haben,
mit Thiophenolen der Formel (VII)

H-S-B (VII)

in welcher
B die oben angegebene Bedeutung hat
in Gegenwart einer Base und gegebenenfalls in Gegenwart eines Verdünnungsmittels umsetzt;
und gegebenenfalls e) die nach den Verfahren (a) bis (d) erhältlichen Verbindungen der Formel (Ia)

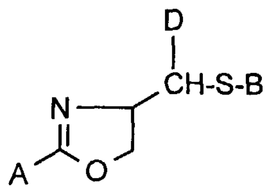

(Ia)

in welcher
A, B und D die oben angegebene Bedeutung haben,
mit einem Oxidationsmittel, gegebenenfalls in Gegenwart eines Verdünnungsmittels und gegebenenfalls in Gegenwart eines Reaktionshilfsmittels umsetzt.

Weiterhin wurde gefunden, daß die Verbindungen der Formel (1) sehr gut zur Bekämpfung von tierischen Schädlingen geeignet sind. Sie zeichnen sich insbesondere durch hohe Wirksamkeit gegen Arthropoden und Nematoden aus.
Überraschenderweise zeigen die erfindungsgemäßen Diphenyloxyzolin-Derivate der Formel (I) eine erheblich bessere Wirksamkeit gegenüber tierischen Schädlingen als die konstitutionell ähnlichsten vorbekannten Verbindungen.
Die erfindungsgemäßen Verbindungen sind durch die Formel (I) allgemein definiert.
Besonders bevorzugte Substituenten bzw. Bereiche der in den oben und nachstehend erwähnten Formeln aufgeführten Reste werden im folgenden erläutert.

A steht besonders bevorzugt für Phenyl, das einfach bis fünffach, gleich oder verschieden substituiert ist durch
F, Cl, Br, $C_1$-$C_3$-Alkyl, $C_1$-$C_3$-Alkoxy, $C_1$-$C_3$-Alkylthio, einfach bis fünffach, gleich oder verschieden durch F und/oder Cl substituiertes $C_1$-$C_2$-Alkyl, einfach bis fünffach, gleich oder verschieden durch F und/oder Cl substituiertes $C_1$-$C_4$-Alkoxy, $SCF_3$ oder $SCHF_2$;

B steht besonders bevorzugt für Phenyl, das gegebenenfalls einfach bis vierfach, gleich oder verschieden substituiert ist durch
F, Cl Br,
$C_1$-$C_{18}$-Alkyl,
Cyano,
$C_1$-$C_6$-Alkoxy-$C_1$-$C_8$-alkyl,
einfach bis sechsfach, gleich oder verschieden durch F und/oder Cl substituiertes $C_1$-$C_8$-Alkoxy,

einfach bis fünffach, gleich oder verschieden durch F und/oder Cl substituiertes $C_1$-$C_2$-Alkyl,
$C_1$-$C_{18}$-Alkoxy und -$(OC_2H_4)_{1-3}$-O-$C_1$-$C_6$-alkyl,
$C_1$-$C_{12}$-Alkylthio,
einfach bis sechsfach, gleich oder verschieden durch F und/oder Cl substituiertes $C_1$-$C_8$-Alkylthio,
3,4-Difluormethylendioxo,
3,4-Tetrafluorethylendioxo,
die Gruppierungen

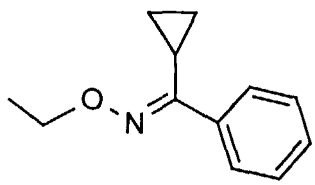 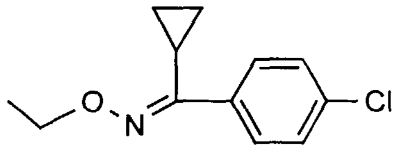 -

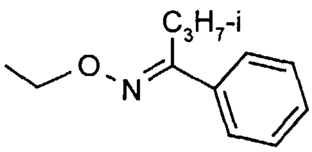 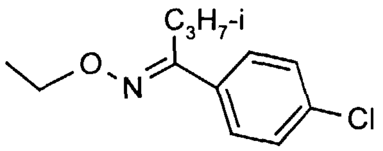 ,

jeweils gegebenenfalls durch $C_1$-$C_4$-Alkyl, $C_1$-$C_4$-Alkoxy, Cyclohexyl oder Phenyl substituiertes Cyclohexyl oder Cyclohexyloxy;
gegebenenfalls einfach oder zweifach, gleich oder verschieden durch F, Cl oder $CF_3$ substituiertes Pyridyloxy;
jeweils gegebenenfalls einfach bis dreifach, gleich oder verschieden durch $C_1$-$C_{12}$-Alkyl, F, Cl, Br, $CF_3$, $C_1$-$C_4$-Alkoxy, einfach bis sechsfach, gleich oder verschieden durch F und/oder Cl substituiertes $C_1$-$C_4$-Alkoxy, $C_1$-$C_4$-Alkoxy-$C_1$-$C_4$-alkyl, $C_1$-$C_4$-Alkoxyethylenoxy, $C_1$-$C_4$-Alkylthio, einfach bis sechsfach, gleich oder verschieden durch F und/oder Cl substituiertes $C_1$-$C_4$-Alkylthio
substituiertes Phenyl, Phenylcarbonyl, Benzyl, Phenoxy, Phenylthio, Benzylthio, Benzyloxy, Benzyloxycarbonyl oder Benzylthio;
$C_1$-$C_{12}$-Alkyl-carbonyl,
$C_1$-$C_{12}$-Alkoxy-carbonyl,
$C_5$-$C_7$-Cycloalkyloxy-carbonyl, das gegebenenfalls durch $C_1$-$C_6$-Alkyl und/oder $C_1$-$C_6$-Alkoxy substituiert ist;
$C_5$-$C_7$-Cycloalkyl-$C_1$-$C_6$-alkyloxy-carbonyl, das gegebenenfalls im Cycloalkylteil durch $C_1$-$C_6$-Alkyl und/oder $C_1$-$C_6$-Alkoxy substituiert ist; und
die Gruppierung -CO-$NY^1Y^2$, wobei

$Y^1$ und $Y^2$ gleich oder verschieden sind und folgende Bedeutungen haben:
Wasserstoff,
$C_1$-$C_6$-Alkyl,
$C_5$-$C_7$-Cycloalkyl, das gegebenenfalls durch $C_1$-$C_6$-Alkyl und/oder
$C_1$-$C_6$-Alkoxy substituiert ist, sowie Benzyl, das gegebenenfalls durch die oben genannten Benzylsubstituenten substituiert ist,

D für Wasserstoff oder Methyl steht und

n für 0, 1 oder 2 steht.

Beispielhaft seien für den Substituenten A folgende Reste genannt:

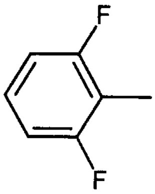 , 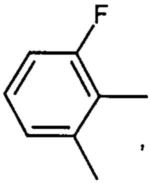 , 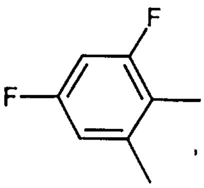 , 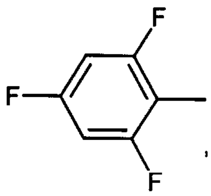 , 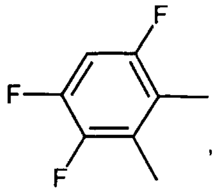 ,

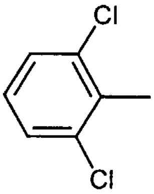 , 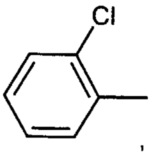 , 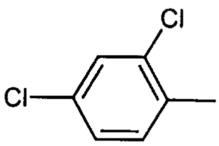 , 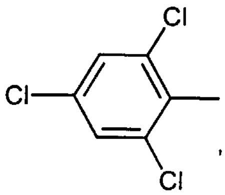 , 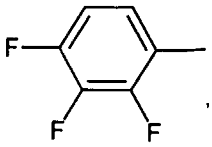 ,

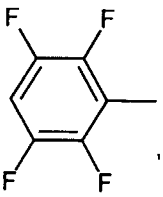 , 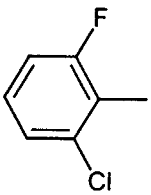 , 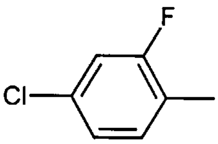 , 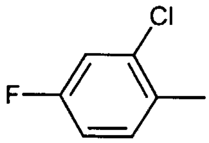 , 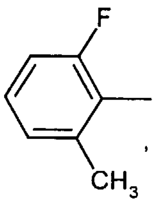 ,

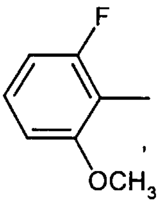 , 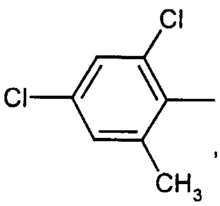 , 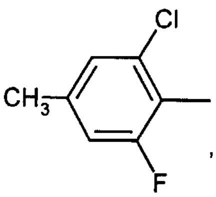 , 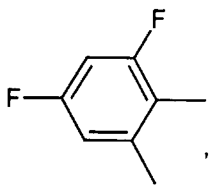 ,

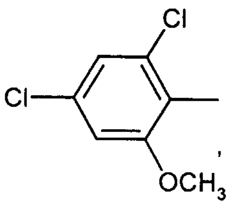 , 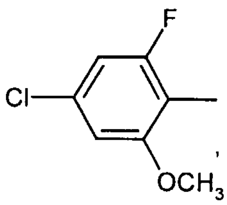 , 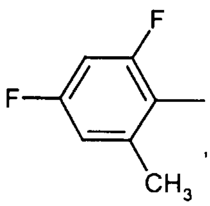 , 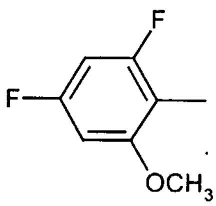 .

Beispielhaft seien für den Substituenten B folgende Reste genannt:

B

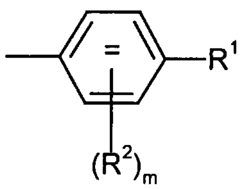

$R^1$ = gemäß Tabelle 1
$(R^2)_m$ = gemäß Tabelle 2

## Tabelle 1

| $R^1$ | $R^1$ |
|---|---|
| Cl | $-OCF_2CHFCF_3$ |
| F | $-CH_2CH_2-O-C_2H_5$ |
| $-C_4H_9$-t | $-CH_2CH_2-O-C_4H_9$-n |
| $-C_6H_{13}$-n | $-CH_2CH_2-O-C_6H_{13}$-n |
| $-C_{12}H_{25}$-n | 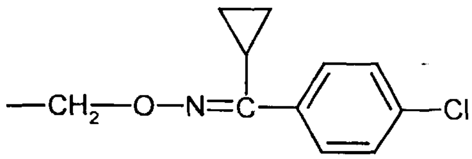 |
| $C_{10}H_{21}$-n | $-SC_4H_9$-n |
| $C_8H_{17}$-n | $-SC_6H_{13}$-n |
| $C_9H_{19}$-n | $-SC_8H_{17}$-n |
| $CF_3$ | $-SC_{12}H_{25}$-n |
| $-CF_2CHF_2$ | $-SCF_3$ |
| $-OC_6H_{13}$-n | $-SCF_2CHF_2$ |
| $-OC_8H_{17}$-n | $-SCF_2CHFCH_3$ |
| $-OC_{12}H_{25}$-n | $-CO-CH_3$ |
| $-OCF_3$ | 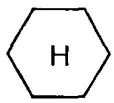 |
| $-OCF_2CHF_2$ | 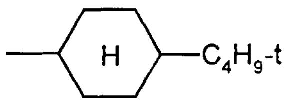 |
| $-OCH_2CF_3$ | 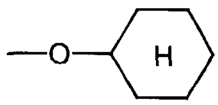 |
| $-OCF_2CHFCH_3$ | 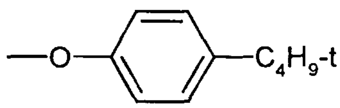 |

| $R^1$ | $R^1$ |
|---|---|
| $-CO-C_4H_9-i$ | 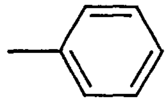 |
| 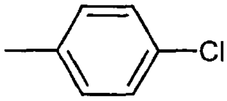 | 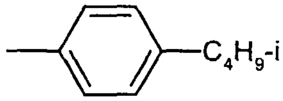 |
| 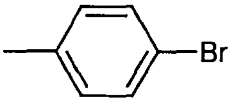 | 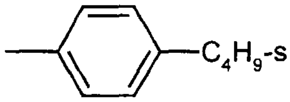 |
| 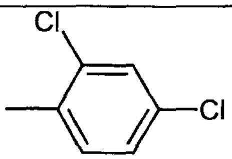 | 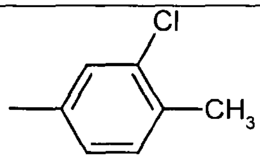 |
| 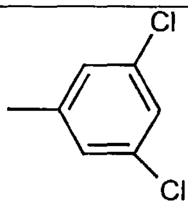 | 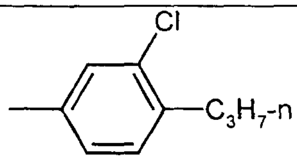 |
| 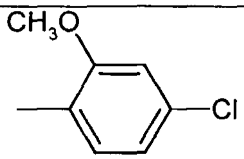 | 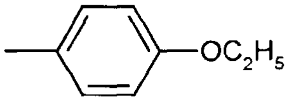 |
| 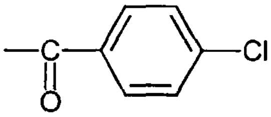 | 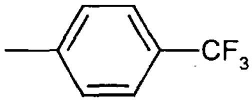 |
| 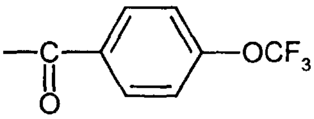 | 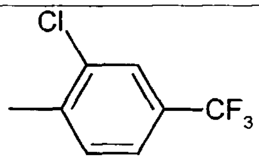 |
| 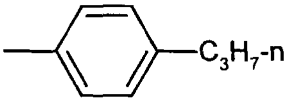 | 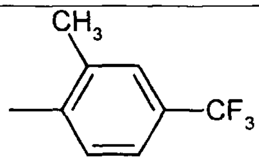 |

| $R^1$ | $R^1$ |
|---|---|
| 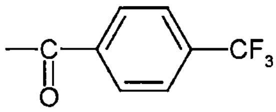 | 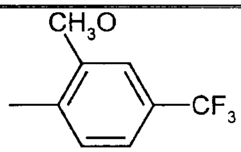 |
| 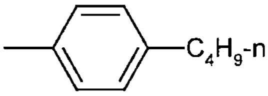 | 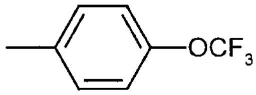 |
| 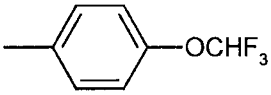 | 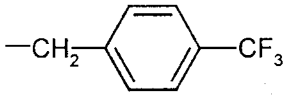 |
| 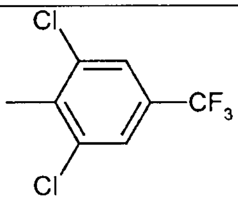 | 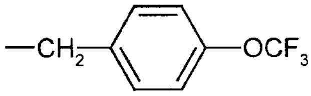 |
| 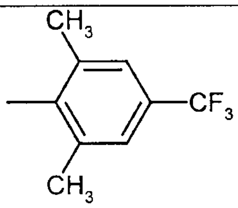 | 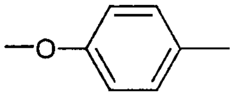 |
| 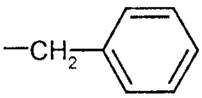 | 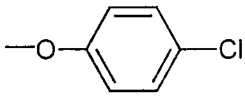 |
| 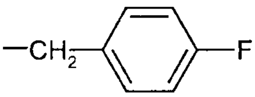 | 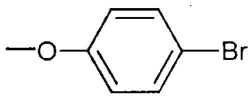 |
| 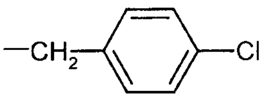 | 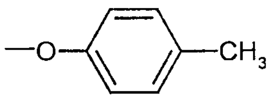 |
| 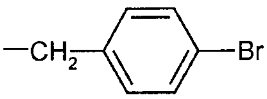 | 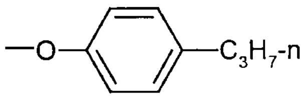 |
| 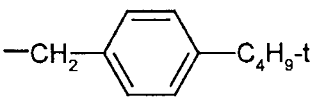 | 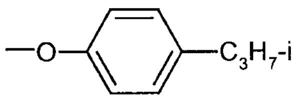 |

| $R^1$ | $R^1$ |
|---|---|
| 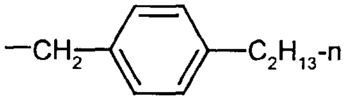 | 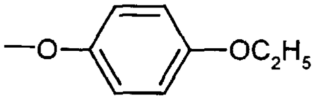 |
| 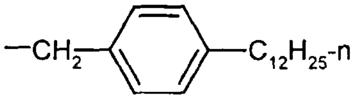 | 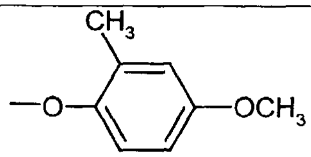 |
| 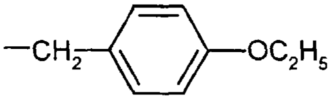 | 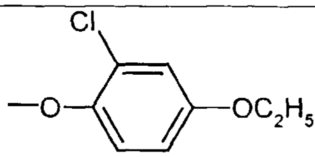 |
| 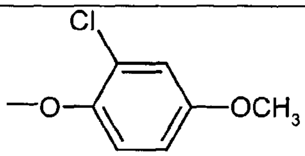 | 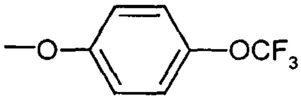 |
| 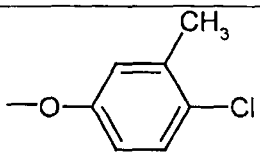 | 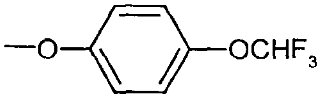 |
| 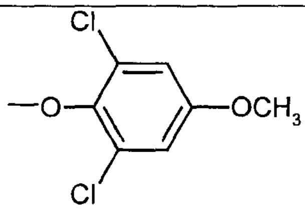 | 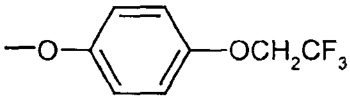 |
| 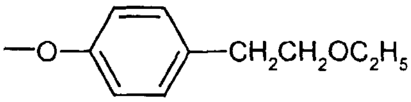 | 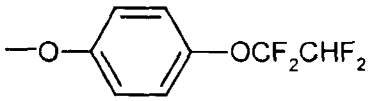 |
| 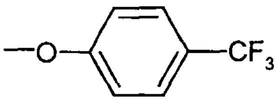 | 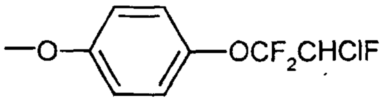 |
| 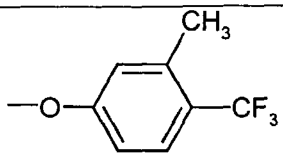 | 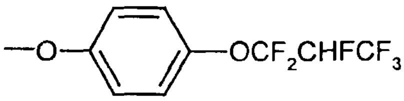 |

| $R^1$ | $R^1$ |
|---|---|
| 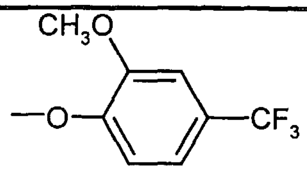 | 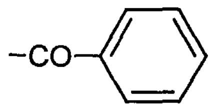 |
| 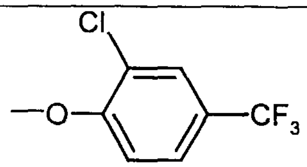 | 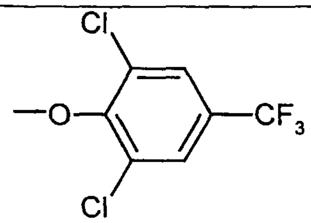 |
| 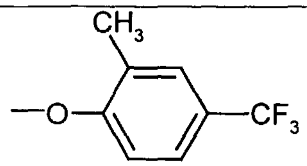 | 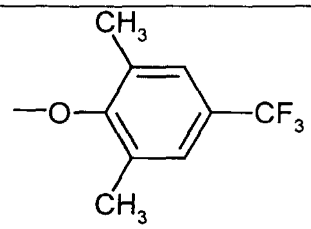 |
| 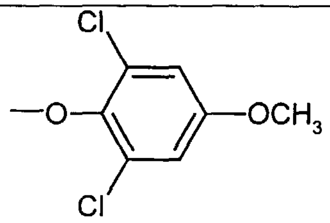 | 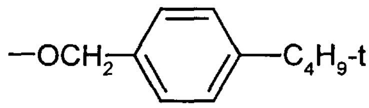 |
| 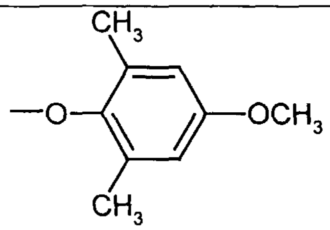 | 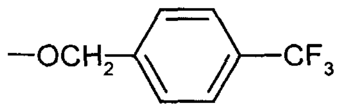 |
| 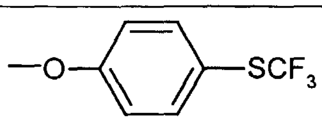 | 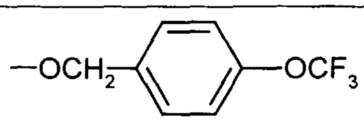 |
| 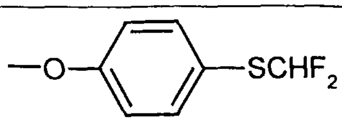 | 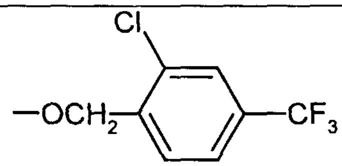 |

| $R^1$ | $R^1$ |
|---|---|
| 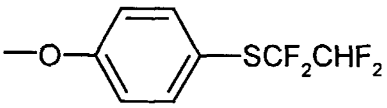 | 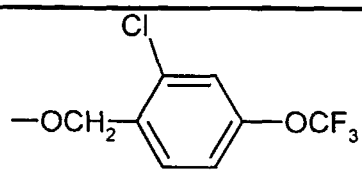 |
| 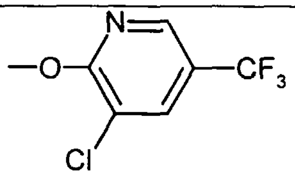 | 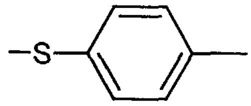 |
| 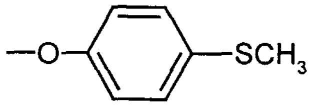 | 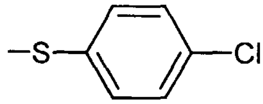 |
| 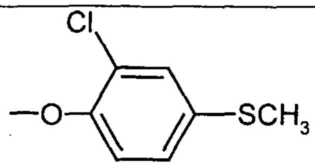 | 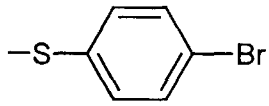 |
| 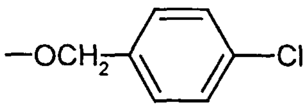 | 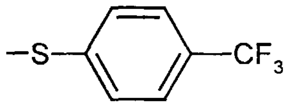 |
| 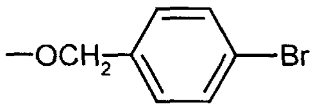 | 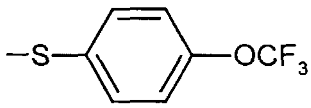 |
| 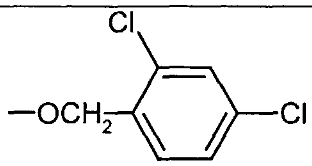 | H |

**Tabelle 2**

| $(R^2)_m$ | $(R^2)_m$ |
|---|---|
| $-CO-O-C_4H_9$-t | $-CO-NH-C_4H_9$-t |
| -CN | |
| H | $2-CH_3$ |
| 2-Cl | $2-OCH_3$ |
| 2-F | $2-Cl_2$ |
| 3-Cl | $2,6-OC_2H_5$ |
| $2,6-Cl_2$ | $3-CH_3$ |
| $3,5-Cl_2$ | $3,5-OCH_3$ |
| $3,5-F_2$ | $3-OC_6H_5$ |
| $2,5-Cl_2$ | |
| $3,5-Cl_2$; 2-F | gemeinsam mit $R^1$ für |
| 2,3 $F_2$ | $3,4-OCF_2O-$ |
| 2,5 $F_2$ | $3,4-OCF_2CF_2O-$ |
| $2,3,5,6-Cl_4$ | 2-Cl; $3-CF_3$ |
| $3-CF_3$ | 2-Cl; $5-CF_3$ |

Im einzelnen seien folgende Beispiele für die erfindungsgemäßen Verbindungen der Formel (Ib) genannt:

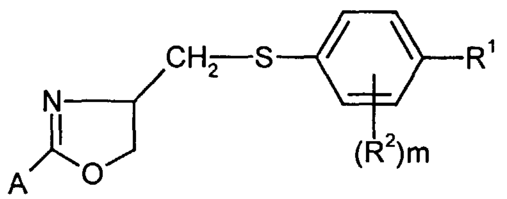

(Ib)

| A | $R^1$ | $(R^2)_m$ |
|---|---|---|
| 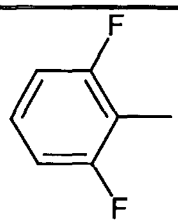 | Cl | 2-Cl, 3-$CF_3$ |
| 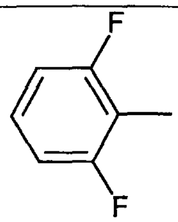 | 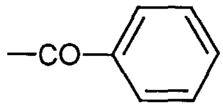 | - |
| 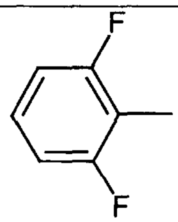 | -CO-OC$(CH_3)_3$ | 2-$CH_3$ |
| 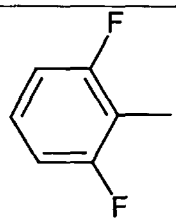 | -C$(CH_3)_3$ | - |
| 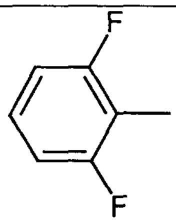 | Cl | 2-Cl, 5-$CF_3$ |

| A | $R^1$ | $(R^2)_m$ |
|---|---|---|
| 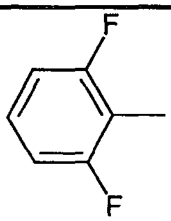 | $-CO-NH-C(CH_3)_3$ | - |
| 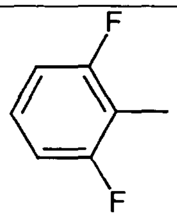 | $-CO-OC_4H_9$-i | - |
| 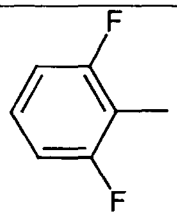 | $-COCH_3$ | 2-Cl |
| 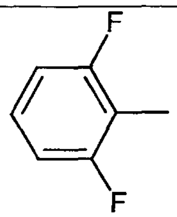 | $-OCF_2-CHF-CF_3$ | 2,5-$Cl_2$ |
| 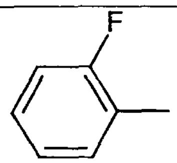 | $-OCF_2-CHF-CF_3$ | 2,5-$Cl_2$ |
| 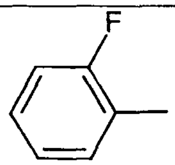 | 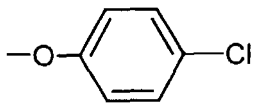 | 3,5-$Cl_2$ |
| 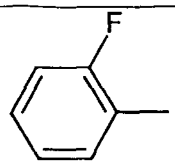 | 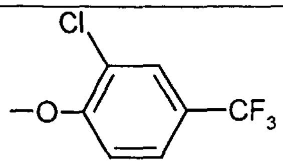 | 2-F |

| A | $R^1$ | $(R^2)_m$ |
|---|---|---|
| 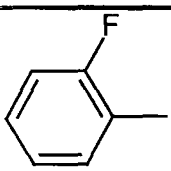 | 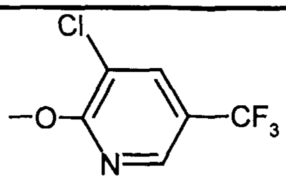 | 3,5-$Cl_2$ |
| 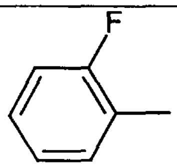 | O-$CF_2$-$CHF_2$ | 3,5-$Cl_2$ |
| 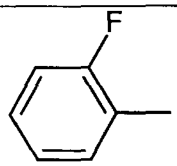 | F | 2-F; 3,5-$Cl_2$ |
| 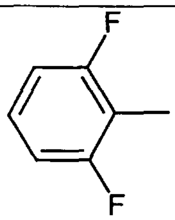 | F | 2-F; 3,5-$Cl_2$ |
| 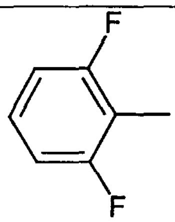 | 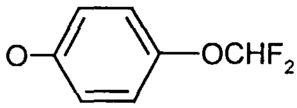 | - |
| 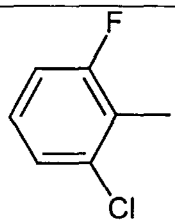 | $CF_3$ | 2,3-$F_2$ |
| 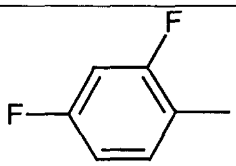 | $CF_3$ | 2,3-$F_2$ |

| A | $R^1$ | $(R^2)_m$ |
|---|---|---|
| 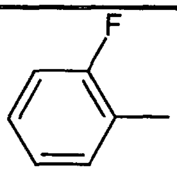 | 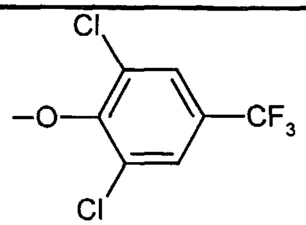 | 2-Cl |
| 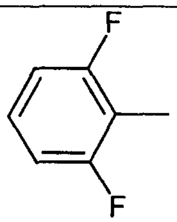 | 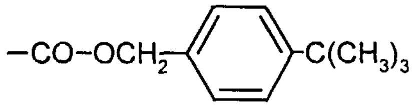 | - |

Verwendet man zur Durchführung des erfindungsgemäßen Verfahrens (a) beispielsweise 2-Amino-3-(4-t-butylphenylthio)-1-propanol und 2,6-Difluorbenzoesäure als Ausgangsstoffe und Polyphosphorsäure als wasserentziehendes Mittel, so kann der Reaktionsablauf durch das folgende Formelschema skizziert werden:

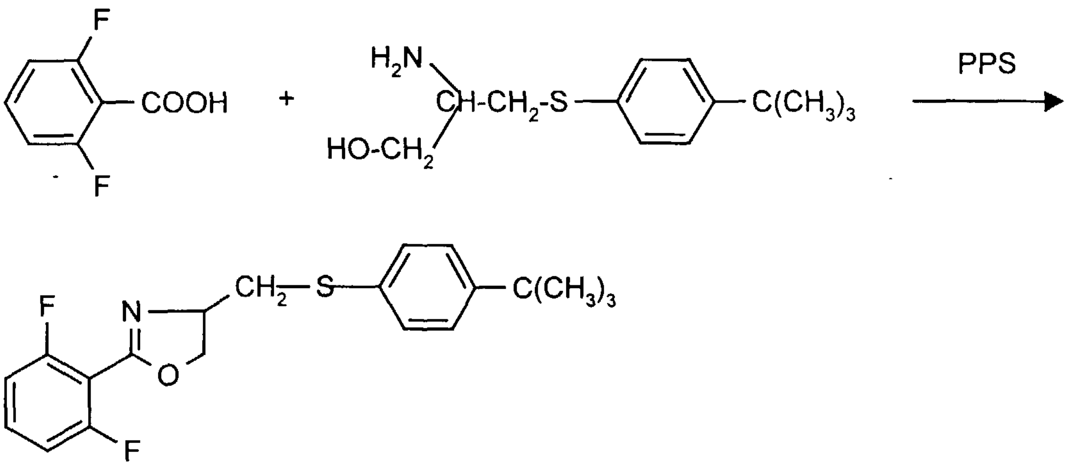


Verwendet man zur Durchführung des erfindungsgemäßen Verfahrens (b) beispielsweise N-[1-Hydroxy-3-(4-t-butylphenylthio)-2-propyl]-2,6-difluorbenzamid als Ausgangsverbindung und Phosphorsäure (PPS) als wasserentziehendes Mittel, so kann der Reaktionsablauf durch das folgende Formelschema skizziert werden:

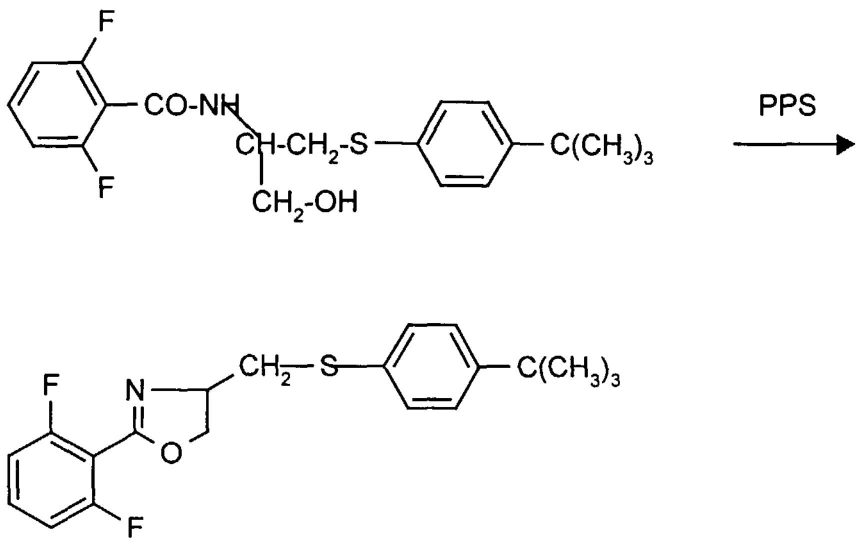


Verwendet man zur Durchführung des erfindungsgemäßen Verfahrens (c) beispielsweise N-[1-Chlor-3-(4-t-butylphenylthio)-2-propyl]-2,6-difluorbenzamid als Ausgangsverbindung und Triethylamin als Base, so kann der Reaktionsablauf durch das folgende Formelschema skizziert werden:

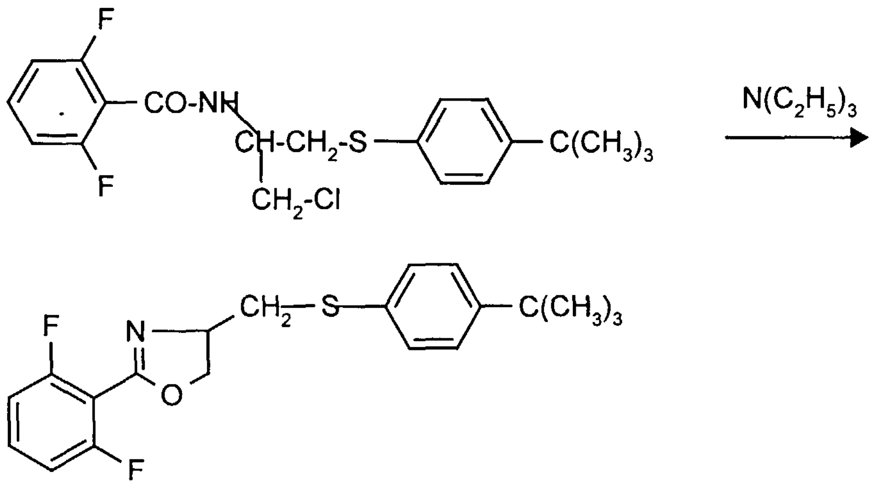


Verwendet man zur Durchführung des erfindungsgemäßen Verfahrens (d) beispielsweise 2-(2,6-Difluorphenyl)-4-(p-tolylsulfonyloxymethyl)-1,3-oxazolin und 4-t-Butyl-thiophenol als Ausgangsstoffe, so kann der Reaktionsablauf durch das folgende Formelschema skizziert werden:

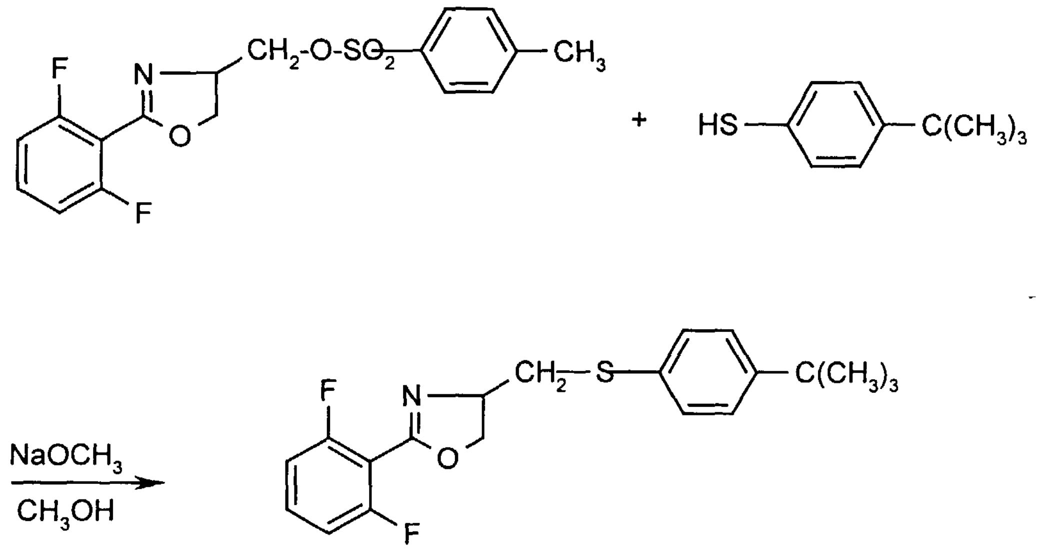


Verwendet man zur Durchführung des erfindungsgemäßen Verfahrens (e) beispielsweise 2-(2,6-Difluorphenyl)-4-(4-t-butylphenylthiomethyl)-1,3-oxazolin als Ausgangsstoff und Wasserstoffperoxid als Oxidationsmittel, so kann der Reaktionsablauf durch das folgende Formelschema skizziert werden:

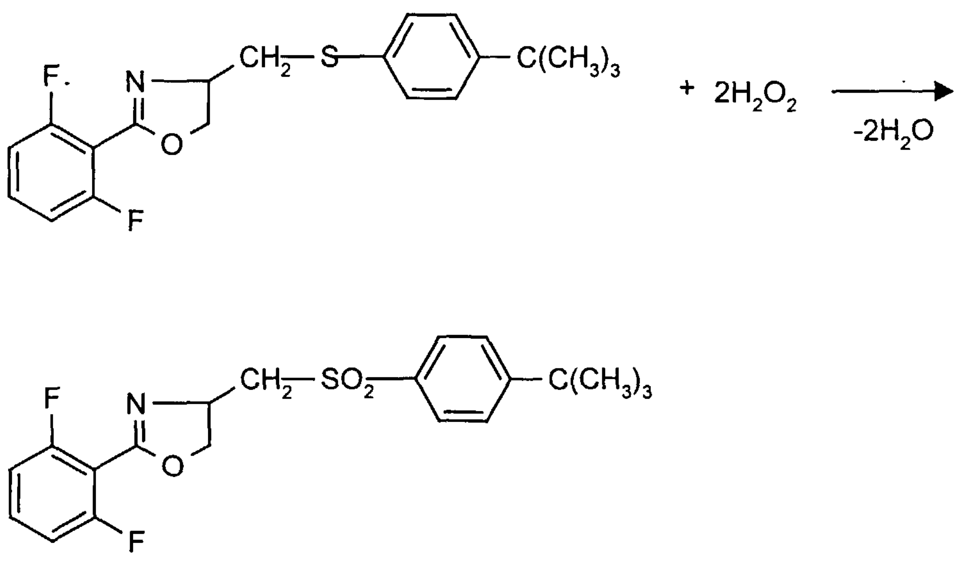


Die beim erfindungsgemäßen Verfahren (a) zur Herstellung der Verbindungen der Formel (I) als Ausgangsstoffe zu verwendenden Aminoalkohole sind durch die Formel (II) allgemein definiert. In der Formel (II) haben B und D vorzugsweise bzw. insbesondere diejenige Bedeutung, die bereits oben im Zusammenhang mit der Beschreibung der Verbindungen der Formel (1) als bevorzugt bzw. als insbesondere bevorzugt für B angegeben wurde.

Die Ausgangsstoffe der Formel (II) sind bekannt und/oder können nach an sich bekannten Verfahren durch Reduktion der entsprechenden Aminosäuren hergestellt werden (vgl. z.B. Chemische Berichte 121 (1988), 2209).

Die beim erfindungsgemäßen Verfahren (a) zur Herstellung der Verbindungen der Formel (I) weiter als Ausgangsstoffe zu verwendenden Carbonsäuren sind durch die Formel (III) allgemein definiert. In der Formel (III) hat A vorzugsweise bzw. insbesondere diejenige Bedeutung, die bereits oben im Zusammenhang mit der Beschreibung der Verbindungen der Formel (I) als bevorzugt bzw. als insbesondere bevorzugt für A angegeben wurde.

Die Ausgangsstoffe der Formel (III) sind bekannte organische Synthesechemikalien.

Die erfindungsgemäßen Verfahren (a) und (b) werden unter Verwendung eines wasser-entziehenden Mittels durchgeführt. Es können die in der organischen Chemie üblichen wasser-entziehenden Mitttel eingesetzt werden. Vorzugsweise verwendbar sind Schwefelsäure, Polyphosphorsäure (PPS), Phosphor(V)-oxid, Dicyclohexylcarbodiimid (DCC), Phosphor(V)-sulfid und das System Triphenylphosphin/Triethylamin/Tetrachlormethan.

Als Verdünnungsmittel zur Durchführung der erfindungsgemäßen Verfahren (a) bis (d) kommen die üblichen organischen Lösungsmittel in Betracht. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid und N,N-Dimethylacetamid, N-Methyl-pyrrolidon oder Hexamethylphosphorsäuretriamid; Ester wie Essigsäuremethylester oder Essigsäureethylester, sowie Sulfoxide, wie Dimethylsulfoxid, gegebenenfalls auch Alkohole wie Methanol oder Ethanol.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 150°C, vorzugsweise bei Temperaturen zwischen 10°C und 100°C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines wasser-entziehenden Mittels durchgeführt, und das Reaktionsgemisch wird mehrere Stunden bei der jeweils erforderlichen Temperatur gerührt. Die Aufarbeitung erfolgt nach üblichen Methoden.

In einer besonderen Ausführungsform des erfindungsgemäßen Verfahrens (a) können statt der Carbonsäuren der Formel (III) auch entsprechende Nitrile eingesetzt werden, wobei dann vorzugsweise an Stelle eines wasser-entziehenden Mittels ein Katalysator, wie z.B. Zink-(II)-chlorid verwendet wird.

Die bei dem erfindungsgemäßen Verfahren (b) zur Herstellung der Verbindungen der Formel (I) als Ausgangsstoffe zu verwendenden Amidalkohole sind durch die Formel (IV) allgemein definiert. In der Formel (IV) haben A, B und D vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, B und D angegeben wurden.

Die Ausgangsstoffe der Formel (IV) sind bekannt und/oder können nach an sich bekannten Verfahren hergestellt werden (vgl. z.B. J. Org. Chem. 53 (1988), 1372; Tetrahedron Lett. 29 (1988), 3121).

Man erhält die Amidalkohole der Formel (IV) beispielsweise, wenn man von den Carbonsäuren der Formel (III) abgeleitete Säurechloride mit Aminoalkoholen der Formel (II) in Gegenwart eines Säurebindemittels, wie z.B. Triethylamin, Pyridin, Kaliumcarbonat oder Natriumhydroxid, und in Gegenwart eines Verdünnungsmittels, wie z.B. Toluol, Chlorbenzol, Aceton oder Acetonitril, bei Temperaturen zwischen 0°C und 100°C umsetzt.

Man erhält die Amidalkohole der Formel (IVa)

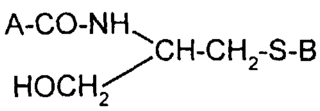

(IVa)

in welcher

A und B die oben angegebene Bedeutung haben,

auch, wenn man
Esteralkoholamide der Formel (XVa)

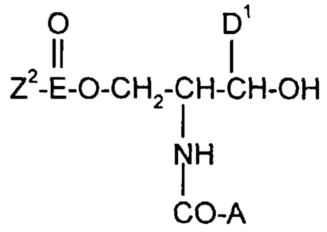

(XVa)

in welcher

A die oben angegebene Bedeutung hat,

$Z^2$ für Alkyl oder gegebenenfalls substituiertes Aryl steht,

E für Kohlenstoff oder SO und

$D^1$ für Wasserstoff steht,

mit Thiophenolen der Formel (VII) in einem geeigneten Lösungsmittel, wie insbesondere Alkoholen, in Gegenwart einer Base, wie insbesondere den entsprechenden Alkalialkoholaten, umsetzt.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +150°C, vorzugsweise bei Temperaturen zwischen 20°C und 140°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (b) zur Herstellung der Verbindungen der Formel (I) setzt man pro Mol an Amidalkohol der Formel (IV) im allgemeinen 1 bis 20 Mol, vorzugsweise 1 bis 5 Mol an wasserentziehendem Mittel ein.

In einer bevorzugten Ausführungsform des erfindungsgemäßen Verfahrens (b) wird der Amidalkohol der Formel (IV) in einem Verdünnungsmittel vorgelegt und das wasser-entziehende Mittel wird dann eindosiert. Das Reaktionsgemisch wird bei der erforderlichen Temperatur bis zum Ende der Umsetzung gerührt und anschließend auf übliche Weise aufgearbeitet.

Die beim erfindungsgemäßen Verfahren (c) zur Herstellung der Verbindungen der Formel (I) als Ausgangsstoffe zu verwendenden Amid-Derivate sind durch die Formel (V) allgemein definiert. In der Formel (V) haben A, B und D vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, B und D angegeben wurden; X steht vorzugsweise für Fluor, Chlor, Brom, Iod, $C_1$-$C_4$-Alkyl-sulfonyloxy, Phenylsulfonyloxy oder Tolylsulfonyloxy, insbesondere für Chlor, Brom, Methylsulfonyloxy oder Tolylsulfonyloxy.

Die Ausgangsstoffe der Formel (V) können nach an sich bekannten Verfahren hergestellt werden.

Man erhält die Amid-Derivate der Formel (V) beispielsweise, wenn man entsprechende Amidalkohole der Formel (IV) mit Chlorierungsmitteln, wie z.B. Thionylchlorid oder Phosphor(V)-chlorid, bzw. mit Sulfonylierungsmitteln, wie z.B. Methansulfonsäurechlorid oder p-Toluolsulfonsäurechlorid in einem Lösungsmittel wie z.B. Methylenchlorid, Toluol oder Essigester in Abwesenheit oder Gegenwart eines Reaktionsbeschleunigers wie z.B. Dimethylformamid oder einer Base bei der Sulfonylierung auf übliche Weise umsetzt.

Man erhält Amid-Derivate der Formel (Va)

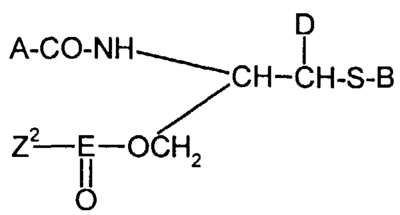

(Va)

in welcher

A, D und B die oben angegebene Bedeutung haben,

E für Kohlenstoff oder SO steht und

$Z^2$ für Alkyl oder gegebenenfalls substituiertes Aryl steht,

indem man Esterhalogenalkylamide der Formel (XVI)

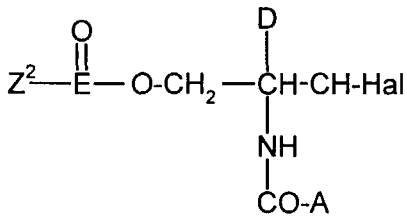

(XVI)

in welcher

A, D, E und $Z^2$ die oben angegebene Bedeutung haben und

Hal für Chlor, Brom oder Iod, insbesondere Chlor oder Brom steht,

mit Thiophenolen der Formel (VII) in einem geeigneten Lösungsmittel, wie insbesondere Alkohole, in Gegenwart einer Base, wie insbesondere den entsprechenden Alkalialkoholaten, umsetzt. Gegebenenfalls kann durch überschüssige Base direkt weiter zum Endprodukt der Formel (I) cyclisiert werden.

Das erfindungsgemäße Verfahren (c) wird in Gegenwart einer Base durchgeführt. Es kommen hierbei alle üblichen anorganischen oder organischen Basen in Betracht. Vorzugsweise verwendbar sind Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +150°C, vorzugsweise bei Temperaturen zwischen 0°C und 100°C.

Das erfindungsgemäße Verfahren (c) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (c) zur Herstellung der Verbindungen der Formel (I) setzt man pro Mol an Amid-Derivat der Formel (V) im allgemeinen 1 bis 3 Mol, vorzugsweise 1,0 bis 1,5 Mol einer Base ein.

In einer bevorzugten Ausführungsform des erfindungsgemäßen Verfahrens (c) werden das Amid-Derivat der Formel (V) und eine Base in einem geeigneten Verdünnungsmittel vermischt; das Gemisch wird bei der erforderlichen Temperatur bis zum Ende der Umsetzung gerührt und anschließend aufübliche Weise aufgearbeitet.

Die beim erfindungsgemäßen Verfahren (d) zur Herstellung der Verbindungen der Formel (I) als Ausgangsstoffe zu verwendenden Oxazoline sind durch die Formel (VI) allgemein definiert. In der Formel (VI) haben A und D vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A und D angegeben wurden. X steht vorzugsweise für Halogen, $C_1$-$C_4$-Alkyl-sulfonyloxy, Phenylsulfonyloxy oder Tolylsulfonyloxy, insbesondere für Methylsulfonyloxy oder Tolylsulfonyloxy.

Die Ausgangsstoffe der Formel (VI) sind bekannt (vgl. z.B. EP-A 0553623) und/oder können z.B. nach den dort beschriebenen Verfahren hergestellt werden.

Sie können auch erhalten werden, indem man z.B. entsprechend dem allgemeinen <u>Reaktionsschema (e):</u>

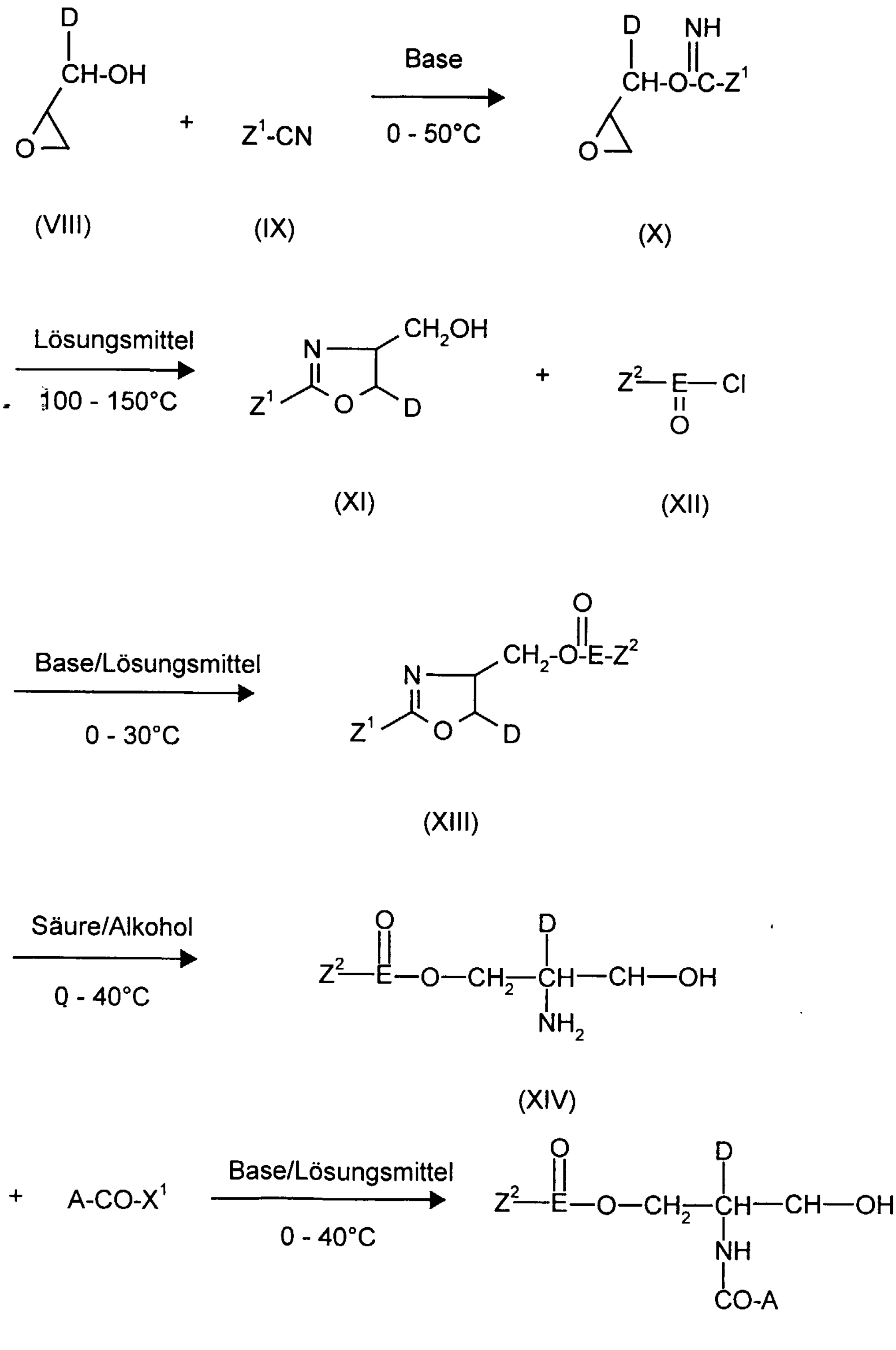


(VIII) (IX) (X)

(XI) (XII)

(XIII)

(XIV)

(IIIa) (XV)

Epoxyalkohole der Formel (VIII, D = Wasserstoff oder Alkyl) mit aktivierten Nitrilen der Formel (IX), in der $Z^1$ für einen ziehenden Rest, wie insbesondere Trichlormethyl steht, in Gegenwart einer Base, wie beispielsweise Kaliumcarbonat, bei 0 - 50°C, vorzugsweise bei 20 - 30°C, zu Iminoesterepoxiden der Formel (X) umsetzt;

diese Iminoesterepoxide der Formel (X) bei 100 - 150°C in einem geeigneten Lösungsmittel, wie beispielsweise Xylol, zu entsprechenden Oxazolinen der Formel (XI) umsetzt;
und diese mit einem Säurechlorid der Formel (XII), in der $Z^2$ für Alkyl oder gegebenenfalls substituiertes Aryl steht und E für Kohlenstoff oder die SO-Gruppe steht, umsetzt (vgl. auch Arch. Pharm. 322, 639 (1989)), wobei diese Acylierung in einem geeigneten Lösungsmittel, wie beispielsweise Essigester, gegebenenfalls in Gegenwart einer Base, wie beispielsweise Triethylamin, bei insbesondere 0 - 30°C durchgeführt wird.

Die entstehenden Oxazoline der Formel (XIII) können mit einer geeigneten, insbesondere anorganischen Säure, wie beispielsweise Salzsäure, in Gegenwart eines Lösungsmittels, wie insbesondere Methanol oder Ethanol, in die entsprechenden Esteralkohole der Formel (XIV) verseift werden;
die über eine entsprechende Acylierung mit aktivierten Derivaten der Carbonsäuren der Formel (III), wie z.B. Säurechloride oder -anhydride der Formel (IIIa; $X^1$ = Chlor oder A-CO-O) zu Esteralkoholamiden der Formel (XV) umgesetzt werden können.

Die Esteramidalkohole der Formel (XV) sind Ausgangsprodukte für die alternativen Verfahrenswege (f) und (g) zur Herstellung von Oxazolinen der Formeln (XVII; beinhaltet Oxazoline der Formel (VI) für D=H) und (XVIII):

Verfahrensweg (f):

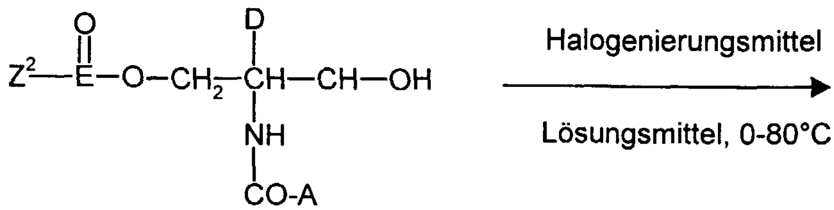


(XV)

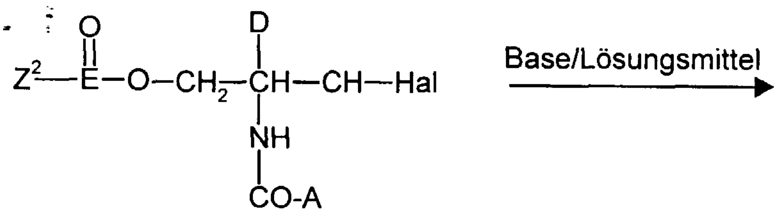


(XVI)

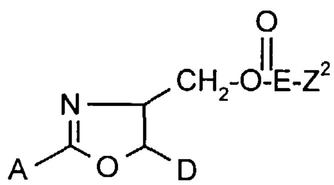

(XVII)

Beim Verfahrensweg (f) wird das Esteralkoholamid der Formel (XV) mit einem geeigneten Halogenierungsmittel, wie z.B. Thionylchlorid, in einem geeigneten Lösungsmittel, wie z.B. Tetrachlorkohlenstoff, in das entsprechende

Esterhalogenalkylamid der Formel (XVI) überführt, das zum Oxazolin der Formel (XVII) cyclisiert wird, wobei gegebenenfalls die Estergruppierung hydrolysiert wird.

Für D=H ist das Oxazolin der Formel (XVII) ein direktes Ausgangsprodukt für das erfindungsgemäße Verfahren (d).

Verfahrensweg (g):

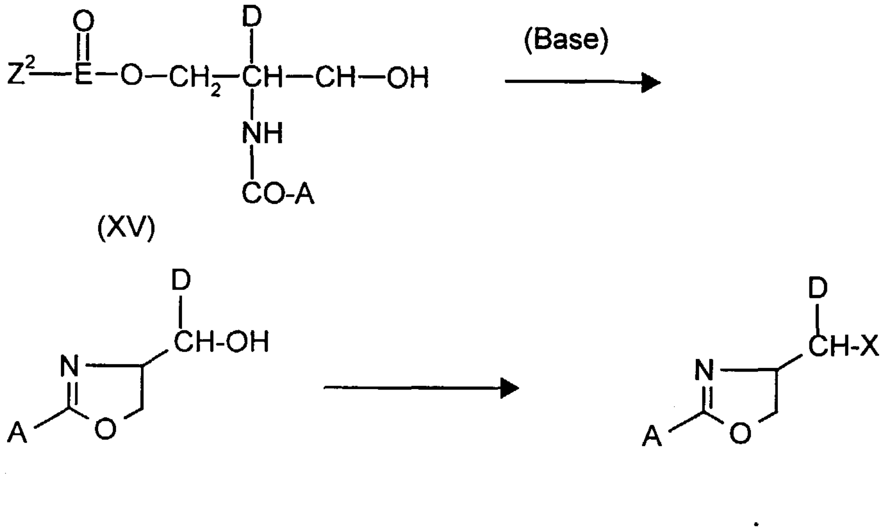


(XV)

(XVIII) (VI)

Beim Verfahrensweg (g) wird das Esteralkoholamid der Formel (XV) gegebenenfalls in Gegenwart einer Base zum Oxazolinalkohol der Formel (XVIII) cyclisiert, der durch Umsetzung mit Sulfonsäurechloriden bzw. mit Halogenierungsmitteln, die für das erfindungsgemäße Verfahren (d) benötigten Oxazoline der Formel (VI) ergibt.

Die beim erfindungsgemäßen Verfahren (d) zur Herstellung der Verbindungen der Formel (I) weiter als Ausgangsstoffe zu verwendenden Thiophenole sind durch die Formel (VII) allgemein definiert. In der Formel (VII) hat B vorzugsweise bzw. insbesondere diejenige Bedeutung, die bereits oben im Zusammenhang mit der Beschreibung der Verbindungen der Formel (I) als bevorzugt bzw. als insbesondere bevorzugt für B angegeben wurde.

Die Ausgangsstoffe der Formel (VII) sind bekannte organische Synthesechemikalien.

Das erfindungsgemäße Verfahren (d) wird in Gegenwart einer Base durchgeführt. Es kommen hierbei alle üblichen anorganischen oder organischen Basen in Betracht. Vorzugsweise verwendbar sind Erdalkali- oder Alkalimetallhydride, -hydroxide, Vorzugsweise verwendbar sind Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen zwischen -20°C und +150°C, vorzugsweise bei Temperaturen zwischen 0°C und 110°C.

Das erfindungsgemäße Verfahren (d) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (d) zur Herstellung der Verbindungen der Formel (I) setzt man pro Mol an Oxazolin der Formel (VI) im allgemeinen 1 bis 3 Mol, vorzugsweise 1 bis 1,5 Mol an Thiophenol der Formel (VII) ein.

In einer bevorzugten Ausführungsform des erfindungsgemäßen Verfahrens (d) werden das Thiophenol der

Formel (VII) und eine Base in einem geeigneten Verdünnungsmittel vorgelegt und tropfenweise mit einem Oxazolin der Formel (VI) in einem geeigneten Verdünnungsmittel versetzt; das Gemisch wird bei der erforderlichen Temperatur bis zum Ende der Umsetzung gerührt und anschließend auf übliche Weise aufgearbeitet.

Die beim erfindungsgemäßen Verfahren (e) zur Herstellung der Verbindungen der Formel (I) als Ausgangsstoffe zu verwendenden Phenylthio-oxazolin-Derivate sind durch die Formel (Ia) allgemein definiert. In der Formel (Ia) haben A, B und D vorzugsweise bzw. insbesondere diejenigen Bedeutungen, die bereits im Zusammenhang mit der Beschreibung der Verbindungen der Formel (I) als bevorzugt bzw. als insbesondere bevorzugt für A, B und D angegeben wurden.

Die Ausgangsstoffe der Formel (Ia) sind erfindungsgemäße Verbindungen und gemäß den Verfahren (a) bis (d) erhältlich.

Als Oxidationsmittel zur Durchführung des erfindungsgemäßen Verfahrens (e) kommen alle üblicherweise für Schwefeloxidationen verwendbaren Oxidationsmittel infrage. Vorzugsweise verwendet man Wasserstoffperoxid oder organische Persäuren, wie beispielsweise Peressigsäure, 4-Nitroperbenzoesäure oder 3-Chlorperbenzoesäure oder auch anorganische Oxidationsmittel, wie Periodsäure, Kaliumpermanganat oder Chromsäure.

Als Verdünnungsmittel zur Durchführung des erfindungsgemäßen Verfahrens (e) kommen in Abhängigkeit vom verwendeten Oxidationsmittel anorganische oder organische Lösungsmittel infrage. Vorzugsweise verwendet man Alkohole, wie Methanol oder Ethanol oder deren Gemische mit Wasser sowie reines Wasser, Säuren oder Säureanhydride, wie beispielsweise Essigsäure, Acetanhydrid oder Propionsäure oder dipolaraprotische Lösungsmittel, wie Acetonitril, Aceton, Essigsäureethylester oder Dimethylformamid sowie auch gegebenenfalls halogenierte Kohlenwasserstoffe, wie Benzin, Benzol, Toluol, Hexan, Cyclohexan, Petrolether, Dichlormethan, Dichlorethan, Chloroform, Tetrachlorkohlenstoff oder Chlorbenzol.

Das erfindungsgemäße Verfahren (e) kann gegebenenfalls in Gegenwart eines Reaktionshilfsmittels durchgeführt werden. Als solche kommen alle üblicherweise verwendbaren organischen und anorganischen Säurebindemittel infrage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (e) kann gegebenenfalls in Gegenwart eines geeigneten Katalysators durchgeführt werden. Als solche kommen alle üblicherweise für derartige Schwefeloxidationen verwendbaren Katalysatoren infrage. Vorzugsweise verwendet man Schwermetallkatalysatoren; beispielsweise genannt seien in diesem Zusammenhang Ammoniummolybdat und Natriumwolframat.

Die Reaktionstemperaturen können bei der Durchführung des erfindungsgemäßen Verfahrens (e) in einem größeren Bereich variiert werden. Im allgemeinen arbeitet man bei Temperaturen zwischen -30°C und +100°C, vorzugsweise bei Temperaturen zwischen 0°C und 80°C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) setzt man pro Mol an Phenylthio-oxazolin-Derivat der Formel (Ia) im allgemeinen 0,8 bis 1,2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an Phenylthio-oxazolin-Derivat der Formel (Ia) im allgemeinen 1,8 bis 3,0 Mol, vorzugsweise doppelt molare Mengen an Oxidationsmittel ein. Die Reaktionsdurchführung, Aufarbeitung und Isolierung der Endprodukte der Formel (I) erfolgt nach üblichen Verfahren.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Verbindungen zeigen in entsprechenden Aufwandmengen auch eine fungizide Wirksamkeit.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgae, wie Halogenkohlenwasserstoffe sowie Butan, Propan, Stickstoff und Kohlendioxid; als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und

Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Genannt seien die folgenden Verbindungen:

Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zeta-methrin, Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb, Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion, Buprofezin, Chlorfluazuron, Diflubenzuron, Flucycloxuron, Flufenoxuron, Hexaflumuron, Pyriproxifen, Tebufenozide, Teflubenzuron, Triflumuron, Imidacloprid, Nitenpyram, N-[(6-Chloro-3-pyridinyl)methyl]-N'-cyano-N-methyl-ethanimidamid (NI-25), Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron, Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox, Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril (AC 303630).

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie

Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Die Herstellung und die Verwendung der erfindungsgemäßen Stoffe werden durch die folgenden Beispiele veranschaulicht:

**Herstellungsbeispiele**

Beispiel 1

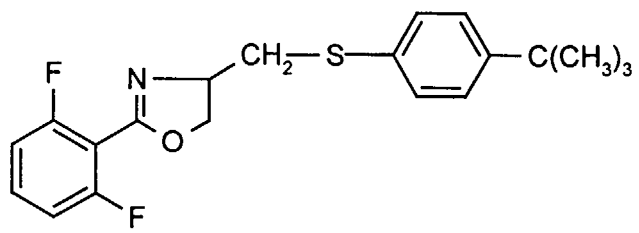

(Verfahren d)

Zu 1 g (6 mMol) 4-t-Butyl-thiophenol in 50 ml Methanol werden unter Rühren 0,3 g (6 mMol) Natriummethylat gegeben (leicht exotherm). Zu dieser Lösung tropft man bei 40°C 2,1 g (6 mMol) 2-(2,6-Difluorphenyl)-4-(p-tolylsulfonyloxymethyl)-1,3-oxazolin in 30 ml Methanol. Man läßt das Reaktionsgemisch 2 Stunden bei 40°C und anschließend 16 Stunden unter Rückfluß rühren. Danach wird das Lösungsmittel abdestilliert, der Rückstand in 100 ml Essigester aufgenommen und dreimal mit je 50 ml Wasser gewaschen. Das Lösungsmittel wird abgezogen und der Rückstand über Kieselgel-Säulenchromatographie mit Toluol:Essigester 10:1 als Laufmittel chromatographiert.

Man erhält 0,9 g (41,6% der Theorie) 2-(2,6-Difluorphenyl)-4-(4-t-butylphenylthiomethyl)-1,3-oxazolin mit einem Verteilungskoeffizienten log P (Octanol/Wasser) von ~4,64 und einem Brechungsindex $n_{20}^{D}$ = 1,5660.

Analog zu Beispiel 1 und gemäß den allgemeinen Angaben zur Herstellung können die nachfolgend aufgeführten Verbindungen der Formel (I) erhalten werden

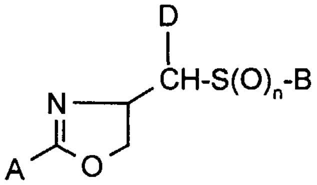

(I)

| Bsp. Nr. | A | D | n | B | physik.Konst. |
|---|---|---|---|---|---|
| 2 | 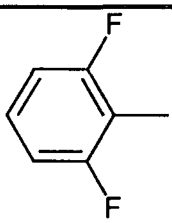 | H | O | 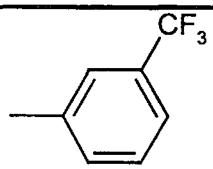 | $n_{20}^{D}$ =1,5433 |
| 3 | 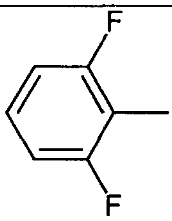 | H | O | 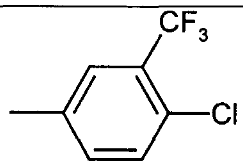 | Fp.: 89-90°C |
| 4 | 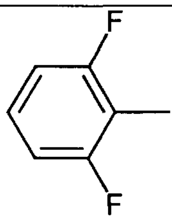 | H | O | 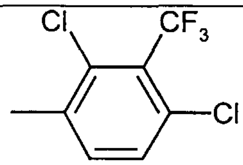 | Fp.: 153-155°C |
| 5 | 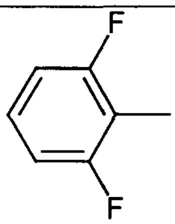 | H | O | 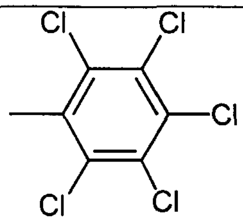 | Fp.:130°C |
| 6 | 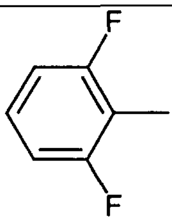 | H | O | 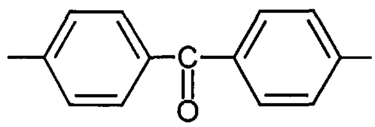 | $n_{20}^{D}$ = 1,6313 |

Fortsetzung

| Bsp. Nr. | A | D | n | B | physik.Konst. |
|---|---|---|---|---|---|
| 7 | 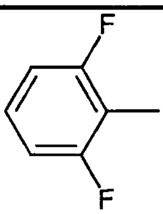 | H | O | 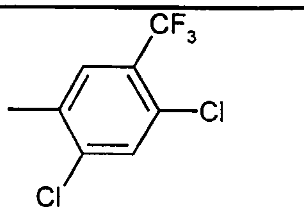 | Fp.: 80-82°C |
| 8 | 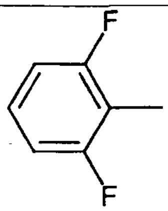 | H | O | 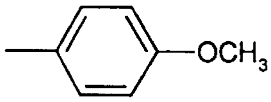 | $n_{20}^{D}$ = 1,5843 |
| 9 | 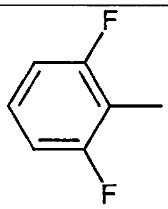 | H | O | 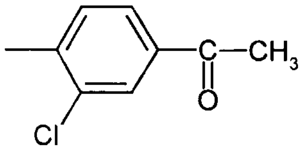 | Fp.: 69-73°C |
| 10 | 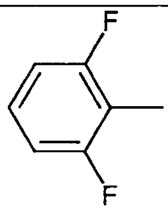 | H | O | 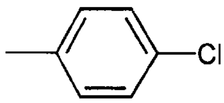 | $n_{20}^{D}$ = 1,5943 |
| 11 | 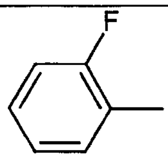 | H | O | 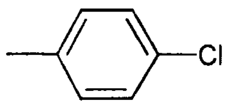 | Fp.: 56°C |
| 12 | 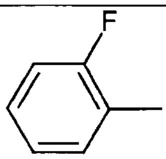 | H | O | 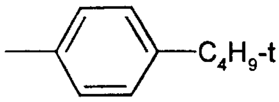 | $n_{20}^{D}$ = 1,5804 |
| 13 | 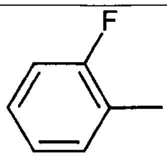 | H | O | 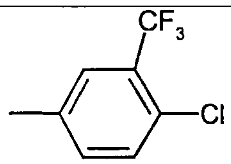 | Fp.: 72°C |

Fortsetzung

| Bsp. Nr. | A | D | n | B | physik.Konst. |
|---|---|---|---|---|---|
| 14 | 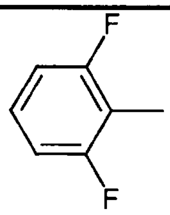 | H | O | 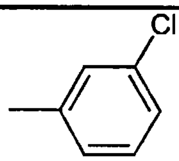 | $n_D^{20}$ = 1,5926 |
| 15 | 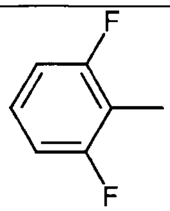 | H | O | 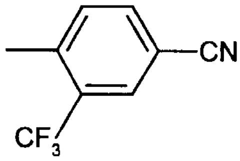 | GC-MS-Spektrum Mol-Peak 398 Hauptpeaks: 182, 154, 127 Nebenpeaks: 379, 351, 326,309, 240, 216, 196 Ret-Index: 2479 |
| 16 | 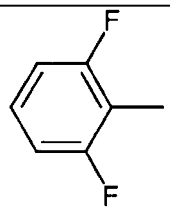 | H | O | 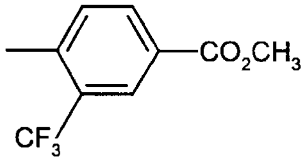 | GC-MS-Spektrum Mol-Peak 431 Hauptpeaks: 182, 154, 127 Nebenpeaks: 412, 400, 384, 342, 250, 219, 204, 196 Ret-Index: 2639 |
| 17 | 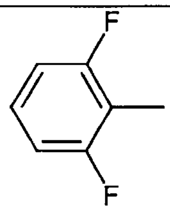 | H | O | 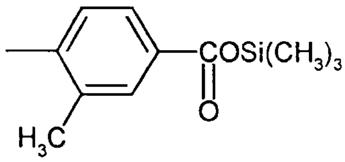 | GC-MS-Spektrum Mol-Peak 435 Hauptpeaks: 182, 196, 154, 141, 127 Nebenpeaks: 420, 388, 363, 346, 317, 278, 254, 239 Ret-Index: 2936 |
| 18 | 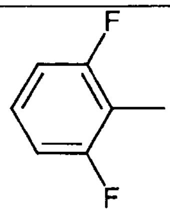 | H | O | 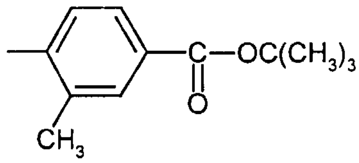 | GC-MS-Spektrum Mol-Peak 419 Hauptpeaks: 182, 196, 154, 141, 127 Nebenpeaks: 386, 372, 346, 262, 223 Ret-Index: 3095 |
| 19 | 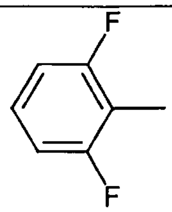 | H | O | 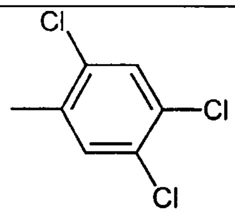 | Fp.: 83°C |

Fortsetzung

| Bsp. Nr. | A | D | n | B | physik.Konst. |
|---|---|---|---|---|---|
| 20 | 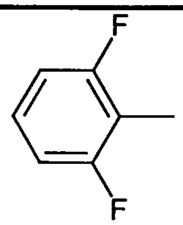 | H | O | 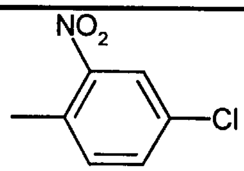 | Fp.: 117°C |
| 21 | 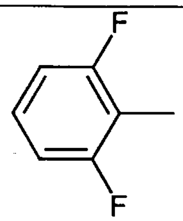 | H | O | 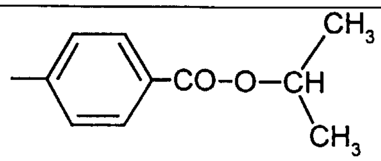 | Log P 3,93 (Octanol/ $H_2O$ |
| 22 | 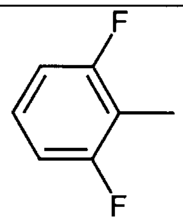 | H | O | 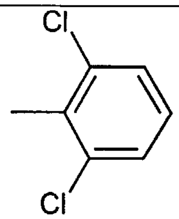 | Fp.: 134°C |
| 23 | 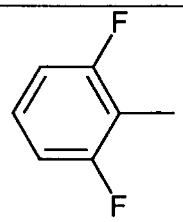 | H | O | 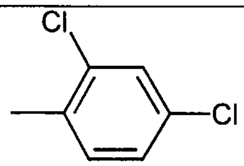 | Fp.: 63°C |
| 24 | 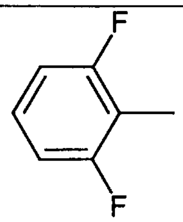 | H | O | 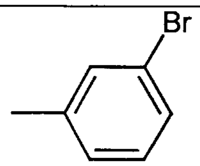 | log P 3.83 |

Herstellung der Ausgangsverbindung

Beispiel (VI-1)

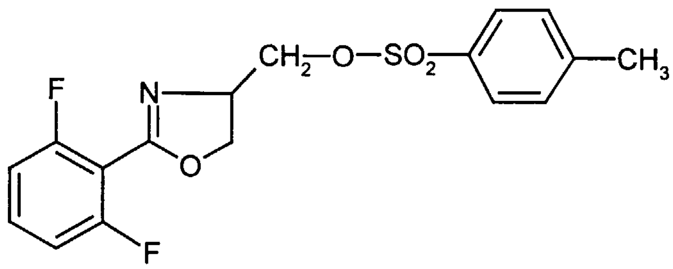

Zu 5,3 g (0,023 Mol) 2-(2,6-Difluorphenyl)-4-hydroxymethyl-1,3-oxazolin in 100 ml Essigester gibt man 5,6 ml (0,069 Mol) Pyridin und tropft anschließend 4,4 g (0,023 Mol) p-Toluolsulfonsäurechlorid in 50 ml Essigester zu. Dabei steigt die Temperatur auf 50°C an. Man rührt 2,5 Stunden bei 50°C und anschließend 8 Stunden unter Rückfluß nach. Die organische Phase wird viermal mit je 50 ml Wasser gewaschen und das Lösungsmittel abdestilliert. Der Rückstand wird über Kieselgel-Säulenchromatographie mit Laufmittel Toluol unter Gradient mit Laufmittel Essigester chromatographiert.

Man erhält 2,1 g (24,8% der Theorie) 2-(2,6-Difluorphenyl)-4-(p-tolylsulfonyloxymethyl)-1,3-oxazolin mit einem Verteilungskoeffizienten log P (Octanol/Wasser) von ~2,68 und einem Brechungsindex $n_{20}^{D}$ = 1,5491.

Beispiel (XVIII-1)

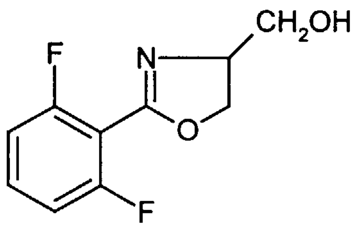

6,5 g (0,0168 Mol) N-[1-Chlor-3-(4-t-butylbenzoyloxy)-2-propyl]-2,6-difluorbenzamid werden in 100 ml Methanol gelöst. Man gibt 2 ml (0,033 Mol) 45%ige Natronlauge zu und läßt 4 Stunden unter Rückfluß rühren. Nach Abdestillieren des Lösungsmittels wird der Rückstand in 100 ml Essigester aufgenommen, dreimal mit je 50 ml Wasser gewaschen und das Lösungsmittel abdestilliert.

Man erhält 2,8 g (72% der Theorie) 2-(2,6-Difluorphenyl)-4-hydroxymethyl-1,3-oxazolin vom Schmelzpunkt 82°C.

Beispiel (XVI-1)

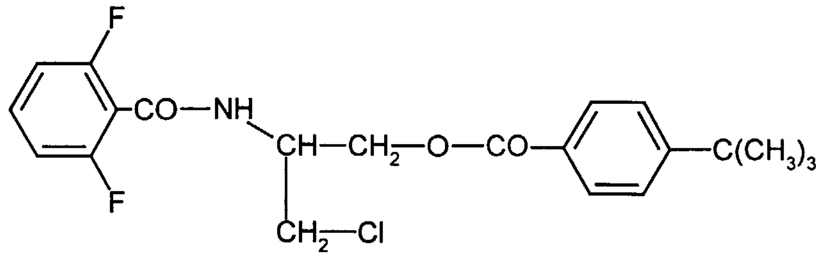

9 g (0,023 Mol) N-[1-Hydroxy-3-(4-t-butylbenzoyloxy)-2-propyl]-2,6-difluorbenzamid werden in 60 ml Tetrachlorkohlenstoff suspendiert, mit 1,7 ml (0,023 Mol) Thionylchlorid versetzt und 1,5 Stunden unter Rückfluß erhitzt. Das Lösungsmittel wird abdestilliert.
Man erhält 9,3 g N-[1-Chlor-3-(4-t-butylbenzoyloxy)-2-propyl]-2,6-difluorbenzamid mit einem Verteilungskoeffizienten log P (Octanol/Wasser) von ~3,86.

Beispiel (XV-1)

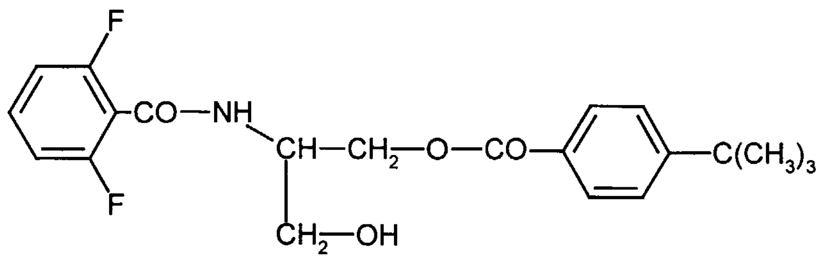

Zu 21 g 2-Amino-3-(4-t-butylbenzoyloxy)-1-propanolhydrochlorid in 400 ml Essigester werden zunächst 21,9 ml (0,158 Mol) Triethylamin zugegeben und anschließend bei 0°C 13,9 g 2,6-Difluorbenzoesäurechlorid in 20 ml Essigester zugetropft. Man läßt 1 Stunde bei 20°C nachrühren; der Niederschlag wird abgesaugt, die organische Phase dreimal mit je 100 ml Wasser gewaschen, das Lösungsmittel abdestilliert und der Rückstand mit Diisopropylether verrührt.
Nach dem Trocknen erhält man 14 g (48% der Theorie) N-[1-Hydroxy-3-(4-t-butylbenzoyloxy)-2-propyl]-2,6-difluorbenzamid mit einem Verteilungskoeffizienten log P (Octanol/Wasser) von 2,82.

Beispiel A

**Tetranychus-Test** (OP-resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe Tetranychus urticae befallen sind, werden mit einer Wirkstoffzubereitung der gewünschten Konzentration gespritzt.

Nach der gewünschten Zeit wird die Wirkung in % bestimmt. Dabei bedeutet 100%, daß alle Spinnmilben abgetötet wurden; 0% bedeutet, daß keine Spinnmilben abgetötet wurden.

In diesem Test bewirkten z.B. die Verbindungen gemäß den Herstellungsbeispielen 2 und 3 bei einer beispielhaften Wirkstoffkonzentration von 0,01 % eine Abtötung von 98 % nach 7 Tagen.

<u>Beispiel B</u>

**Phaedon-Larven-Test**

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven Phaedon cochleariae besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigt z.B. die Verbindung des Herstellungsbeispiels 1 bei einer Wirkstoffkonzentration von 0,1% nach 7 Tagen einen Abtötungsgrad von 100%.

<u>Beispiel C</u>

**Plutella-Test**

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkte z.B. die Verbindung gemäß Herstellungsbeispiel 2 bei einer beispielhaften Wirkstoffkonzentration von 0,1 % eine Abtötung von 100 % nach 7 Tagen.

<u>Beispiel D</u>

**Nephotettix-Test**

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser

auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen 2, 7 und 8 bei einer beispielhaften Wirkstoffkonzentration von 0,1 % eine Abtötung von 100 % nach 6 Tagen.

## Patentansprüche

1. Verbindungen der Formel (I)

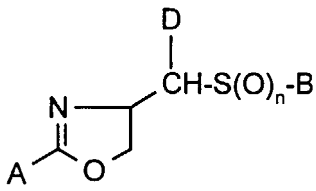

(I)

in welcher

A für Phenyl steht, das einfach bis fünffach, gleich oder verschieden substituiert ist durch
Halogen,
$C_1$-$C_6$-Alkyl,
$C_1$-$C_6$-Alkoxy,
$C_1$-$C_6$-Alkylthio,
$C_1$-$C_6$-Halogenalkyl,
$C_1$-$C_6$-Halogenalkoxy oder $C_1$-$C_6$-Halogenalkylthio;

B für Phenyl steht, das gegebenenfalls einfach bis vierfach, gleich oder verschieden substituiert ist durch
Halogen,
$C_1$-$C_{18}$-Alkyl,
Cyano,
$C_1$-$C_8$-Alkoxy-$C_1$-$C_8$-alkyl,
$C_1$-$C_8$-Halogenalkoxy,
$C_1$-$C_4$-Halogenalkyl,
$C_1$-$C_{18}$-Alkoxy, das gegebenenfalls durch weitere 1-3 Sauerstoffatome unterbrochen ist,
$C_1$-$C_{18}$-Alkylthio,
$C_1$-$C_8$-Halogenalkylthio,
3,4-Difluormethylendioxo,
3,4-Tetrafluorethylendioxo,
gegebenenfalls durch $C_1$-$C_4$-Alkyl, $C_3$-$C_6$-Cycloalkyl und/oder Halogen substituiertes Benzyliminooxymethyl,
jeweils gegebenenfalls durch $C_1$-$C_6$-Alkyl, $C_1$-$C_6$-Alkoxy, Cyclohexyl oder Phenyl substituiertes Cyclohexyl oder Cyclohexyloxy;
gegebenenfalls einfach oder zweifach, gleich oder verschieden durch Halogen, $C_1$-$C_4$-Alkyl oder $C_1$-$C_4$-Halogenalkyl substituiertes Pyridyloxy;
jeweils gegebenenfalls einfach bis dreifach, gleich oder verschieden durch $C_1$-$C_{12}$-Alkyl, Halogen, $C_1$-$C_4$-Halogenalkyl, $C_1$-$C_6$-Alkoxy, $C_1$-$C_6$-Halogenalkoxy, $C_1$-$C_6$-Alkoxy-$C_1$-$C_6$-alkyl, $C_1$-$C_6$-Alkoxyethylenoxy, $C_1$-$C_6$-Alkylthio und/oder $C_1$-$C_6$-Halogenalkylthio substituiertes Phenyl, Phenylcarbonyl, Benzyl, Phenoxy, Phenylthio, Benzyloxy, Benzyloxycarbonyl oder Benzylthio;
$C_1$-$C_{12}$-Alkyl-carbonyl,
$C_1$-$C_{12}$-Alkoxy-carbonyl,
$C_5$-$C_7$-Cycloalkyloxy-carbonyl, das gegebenenfalls durch $C_1$-$C_6$-Alkyl und/oder $C_1$-$C_6$-Alkoxy substituiert ist;
$C_5$-$C_7$-Cycloalkyl-$C_1$-$C_6$-alkyloxy-carbonyl, das gegebenenfalls im Cycloalkylteil durch $C_1$-$C_6$-Alkyl und/oder $C_1$-$C_6$-Alkoxy substituiert ist; und

die Gruppierung -CO-$NY^1Y^2$, wobei
$Y^1$ und $Y^2$ gleich oder verschieden sind und folgende Bedeutungen haben:
Wasserstoff,
$C_1$-$C_6$-Alkyl,
$C_5$-$C_7$-Cycloalkyl, das gegebenenfalls durch $C_1$-$C_6$-Alkyl und/oder $C_1$-$C_6$-Alkoxy substituiert ist, sowie Benzyl, das gegebenenfalls durch die o.g. Benzylsubstituenten substituiert ist,

D für Wasserstoff oder Methyl steht und

n für 0, 1 oder 2 steht.

**2.** Verbindungen der Formel (I) gemäß Anspruch 1, in welcher

A für Phenyl steht, das einfach bis fünffach, gleich oder verschieden substituiert ist durch
F, Cl, Br, $C_1$-$C_3$-Alkyl, $C_1$-$C_3$-Alkoxy, $C_1$-$C_3$-Alkylthio, einfach bis fünffach, gleich oder verschieden durch F und/oder Cl substituiertes $C_1$-$C_2$-Alkyl, einfach bis fünffach, gleich oder verschieden durch F und/oder Cl substituiertes $C_1$-$C_4$-Alkoxy, $SCF_3$ oder $SCHF_2$;

B für Phenyl steht, das gegebenenfalls einfach bis vierfach, gleich oder verschieden substituiert ist durch
F, Cl Br,
$C_1$-$C_{18}$-Alkyl,
Cyano,
$C_1$-$C_6$-Alkoxy-$C_1$-$C_8$-alkyl,
einfach bis sechsfach, gleich oder verschieden durch F und/oder Cl substituiertes $C_1$-$C_8$-Alkoxy,
einfach bis fünffach, gleich oder verschieden durch F und/oder Cl substituiertes $C_1$-$C_2$-Alkyl,
$C_1$-$C_{18}$-Alkoxy und -$(OC_2H_4)_{1-3}$-O-$C_1$-$C_6$-alkyl,
$C_1$-$C_{12}$-Alkylthio,
einfach bis sechsfach, gleich oder verschieden durch F und/oder Cl substituiertes $C_1$-$C_8$-Alkylthio,
3,4-Difluormethylendioxo,
3,4-Tetrafluorethylendioxo,
die Gruppierungen

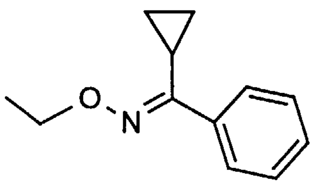
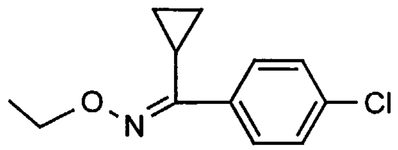
-
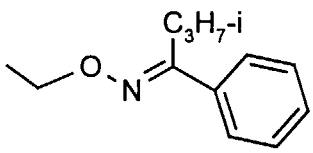
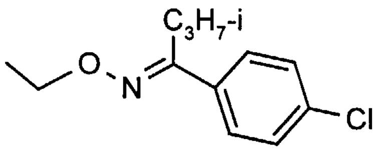
,

jeweils gegebenenfalls durch $C_1$-$C_4$-Alkyl, $C_1$-$C_4$-Alkoxy, Cyclohexyl oder Phenyl substituiertes Cyclohexyl oder Cyclohexyloxy;
gegebenenfalls einfach oder zweifach, gleich oder verschieden durch F, Cl oder $CF_3$ substituiertes Pyridyloxy;
jeweils gegebenenfalls einfach bis dreifach, gleich oder verschieden durch
$C_1$-$C_{12}$-Alkyl, F, Cl, Br, $CF_3$, $C_1$-$C_4$-Alkoxy, einfach bis sechsfach, gleich oder verschieden durch F und/oder Cl substituiertes $C_1$-$C_4$-Alkoxy, $C_1$-$C_4$-Alkoxy-$C_1$-$C_4$-alkyl, $C_1$-$C_4$-Alkoxyethylenoxy, $C_1$-$C_4$-Alkylthio, einfach bis sechsfach, gleich oder verschieden durch F und/oder Cl substituiertes $C_1$-$C_4$-Alkylthio,
substituiertes Phenyl, Phenylcarbonyl, Benzyl, Phenoxy, Phenylthio, Benzylthio, Benzyloxy, Benzyloxycarbonyl oder Benzylthio;
$C_1$-$C_{12}$-Alkyl-carbonyl,
$C_1$-$C_{12}$-Alkoxy-carbonyl,

$C_5$-$C_7$-Cycloalkyloxy-carbonyl, das gegebenenfalls durch $C_1$-$C_6$-Alkyl und/oder $C_1$-$C_6$-Alkoxy substituiert ist; $C_5$-$C_7$-Cycloalkyl-$C_1$-$C_6$-alkyloxy-carbonyl, das gegebenenfalls im Cycloalkylteil durch $C_1$-$C_6$-Alkyl und/oder $C_1$-$C_6$-Alkoxy substituiert ist; und
die Gruppierung $-CO-NY^1Y^2$, wobei
$Y^1$ und $Y^2$ gleich oder verschieden sind und folgende Bedeutungen haben:
Wasserstoff,
$C_1$-$C_6$-Alkyl,
$C_5$-$C_7$-Cycloalkyl, das gegebenenfalls durch $C_1$-$C_6$-Alkyl und/oder $C_1$-$C_6$-Alkoxy substituiert ist, sowie Benzyl, das gegebenenfalls durch die oben genannten Benzylsubstituenten substituiert ist,

D für Wasserstoff oder Methyl steht und

n für 0, 1 oder 2 steht.

**3.** Verfahren zur Herstellung von Verbindungen der Formel (I) gemäß Anspruch 1, dadurch gekennzeichnet, daß man

a) Aminalkohole der Formel (II)

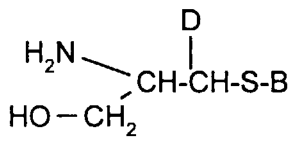

(II)

in welcher
B und D die in Anspruch 1 angegebene Bedeutung haben,
mit einer Carbonsäure der Formel (III)

A-COOH (III)

in welcher

A die in Anspruch 1 angegebene Bedeutung hat,

mit einem wasser-entziehenden Mittel gegebenenfalls in Gegenwart eines Verdünnungsmittels umsetzt; oder

b) Amidalkohole der Formel (IV)

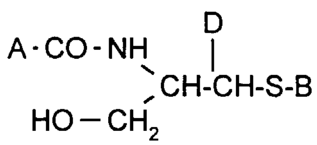

(IV)

in welcher
A, B und D die oben angegebene Bedeutung haben,
mit einem wasser-entziehenden Mittel, gegebenenfalls in Gegenwart eines Verdünnungsmittels umsetzt; oder

c) Amid-Derivate der Formel (V)

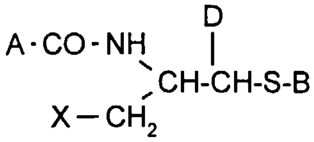

(V)

in welcher
A, B und D die oben angegebene Bedeutung haben; und

X für eine Abgangsgruppe, wie Halogen, Alkylsulfonyloxy oder Arylsulfonyloxy steht,

mit einer Base, gegebenenfalls in Gegenwart eines Verdünnungsmittels umsetzt;
oder

d) Oxazoline der Formel (VI)

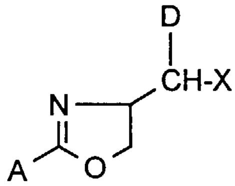

(VI)

in welcher
A, D und X die oben angegebene Bedeutung haben,
mit Thiophenolen der Formel (VII)

H-S-B (VII)

in welcher

B die oben angegebene Bedeutung hat

in Gegenwart einer Base und gegebenenfalls in Gegenwart eines Verdünnungsmittels umsetzt;
und gegebenenfalls

e) die nach den Verfahren (a) bis (d) erhältlichen Verbindungen der Formel (Ia)

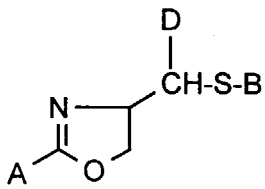

(Ia)

in welcher
A, B und D die oben angegebene Bedeutung haben,
mit einem Oxidationsmittel, gegebenenfalls in Gegenwart eines Verdünnungsmittels und gegebenenfalls in Gegenwart eines Reaktionshilfsmittels umsetzt.

**4.** Schädlingsbekämpfungsmittel, gekennzeichnet durch einen Gehalt an mindestens einer Verbindung der Formel (I) gemäß Anspruch 1.

**5.** Verfahren zur Bekämpfung von tierischen Schädlingen, dadurch gekennzeichnet, daß man Verbindungen der Formel (I) gemäß Anspruch 1 auf tierische Schädlinge und/oder deren Lebensraum einwirken läßt.

**6.** Verwendung von Verbindungen der Formel (I) gemäß Anspruch 1 zur Bekämpfung von tierischen Schädlingen.

**7.** Verfahren zur Herstellung von Schädlingsbekämpfungsmitteln, dadurch gekennzeichnet, daß man Verbindungen der Formel (I) gemäß Anspruch 1 mit Streckmitteln und/oder oberflächenaktiven Mitteln vermischt.

**Claims**

**1.** Compounds of the formula (I)

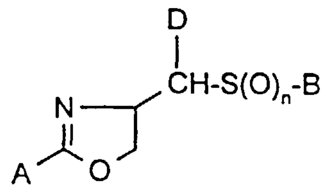

(I)

in which

A represents phenyl which is monosubstituted to pentasubstituted by identical or different substituents from the series consisting of halogen,
$C_1$-$C_6$-alkyl,
$C_1$-$C_6$-alkoxy,
$C_1$-$C_6$-alkylthio,
$C_1$-$C_6$-halogenoalkyl,
$C_1$-$C_6$-halogenoalkoxy or $C_1$-$C_6$-halogenoalkylthio; B represents phenyl which is optionally monosubstituted to tetrasubstituted by identical or different substituents from the series consisting of halogen,
$C_1$-$C_{18}$-alkyl,
cyano,
$C_1$-$C_8$-alkoxy-$C_1$-$C_8$-alkyl,
$C_1$-$C_8$-halogenoalkoxy,
$C_1$-$C_4$-halogenoalkyl,
$C_1$-$C_{18}$-alkoxy which is optionally interrupted by a further 1-3 oxygen atoms,
$C_1$-$C_{18}$-alkylthio,
$C_1$-$C_8$-halogenoalkylthio,
3,4-difluoromethylenedioxo,
3,4-tetrafluoroethylenedioxo,
benzyliminooxymethyl which is optionally substituted by $C_1$-$C_4$-alkyl, $C_3$-$C_6$-cycloalkyl and/or halogen,
cyclohexyl or cyclohexyloxy, which is optionally substituted by $C_1$-$C_6$-alkyl, $C_1$-$C_6$-alkoxy, cyclohexyl or phenyl;
pyridyloxy which is optionally monosubstituted or disubstituted by identical or different substituents from the series consisting of halogen, $C_1$-$C_4$-alkyl or $C_1$-$C_4$-halogenoalkyl;
phenyl, phenylcarbonyl, benzyl, phenoxy, phenylthio, benzyloxy, benzyloxycarbonyl or benzylthio, each of which is optionally monosubstituted to trisubstituted by identical or different substituents from the series consisting of $C_1$-$C_{12}$-alkyl, halogen, $C_1$-$C_4$-halogenoalkyl, $C_1$-$C_6$-alkoxy, $C_1$-$C_6$-halogenoalkoxy, $C_1$-$C_6$-alkoxy-$C_1$-$C_6$-alkyl, $C_1$-$C_6$-alkoxy-ethyleneoxy, $C_1$-$C_6$-alkylthio and/or $C_1$-$C_6$-halogenoalkylthio;
$C_1$-$C_{12}$-alkyl-carbonyl,
$C_1$-$C_{12}$-alkoxy-carbonyl,
$C_5$-$C_7$-cycloalkyloxy-carbonyl which is optionally substituted by $C_1$-$C_6$-alkyl and/or $C_1$-$C_6$-alkoxy;
$C_5$-$C_7$-cycloalkyl-$C_1$-$C_6$-alkyloxy-carbonyl which is optionally substituted in the cycloalkyl moiety by $C_1$-$C_6$-alkyl and/or $C_1$-$C_6$-alkoxy; and
the group $-CO-NY^1Y^2$ in which
$Y^1$ and $Y^2$ are identical or different and have the following meanings:
hydrogen,

$C_1$-$C_6$-alkyl,
$C_5$-$C_7$-cycloalkyl which is optionally substituted by $C_1$-$C_6$-alkyl and/or $C_1$-$C_6$-alkoxy, and
benzyl which is optionally substituted by the abovementioned benzyl substituents,

D represents hydrogen or methyl and

n represents 0, 1 or 2.

**2.** Compounds of the formula (I) according to Claim 1, in which

A represents phenyl which is monosubstituted to pentasubstituted by identical or different substituents from the series consisting of
F, Cl, Br, $C_1$-$C_3$-alkyl, $C_1$-$C_3$-alkoxy, $C_1$-$C_3$-alkylthio, $C_1$-$C_2$-alkyl which is monosubstituted to pentasubstituted by identical or different substituents from the series consisting of F and Cl, $C_1$-$C_4$-alkoxy which is monosubstituted to pentasubstituted by identical or different substituents from the series consisting of F and Cl, $SCF_3$ or $SCHF_2$;

B represents phenyl which is optionally monosubstituted to tetrasubstituted by identical or different substituents from the series consisting of
F, Cl, Br,
$C_1$-$C_{18}$-alkyl,
cyano,
$C_1$-$C_6$-alkoxy-$C_1$-$C_8$-alkyl,
$C_1$-$C_8$-alkoxy which is monosubstituted to hexasubstituted by identical or different substituents from the series consisting of F and Cl,
$C_1$-$C_2$-alkyl which is monosubstituted to pentasubstituted by identical or different substituents from the series consisting of F and/or Cl,
$C_1$-$C_{18}$-alkoxy and $-(OC_2H_4)_{1-3}$-O-$C_1$-$C_6$-alkyl,
$C_1$-$C_{12}$-alkylthio,
$C_1$-$C_8$-alkylthio which is monosubstituted to hexasubstituted by identical or different substituents from the series consisting of F and Cl,
3,4-difluoromethylenedioxo,
3,4-tetrafluoroethylenedioxo,
the groups

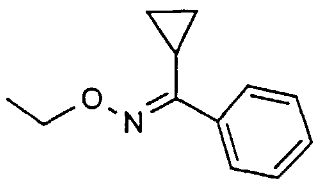 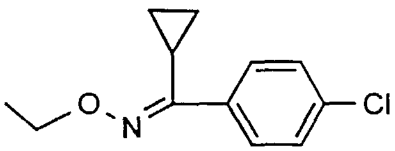 -

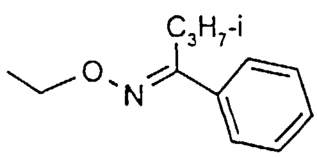 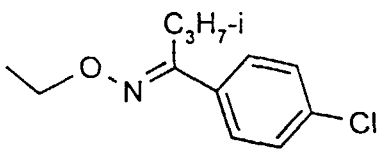 ,

cyclohexyl or cyclohexyloxy, each of which is optionally substituted by $C_1$-$C_4$-alkyl, $C_1$-$C_4$-alkoxy, cyclohexyl or phenyl;
pyridyloxy which is optionally monosubstituted or disubstituted by identical or different substituents from the series consisting of F, Cl or $CF_3$, phenyl, phenylcarbonyl, benzyl, phenoxy, phenylthio, benzylthio, benzyloxy, benzyloxycarbonyl or benzylthio, each of which is optionally monosubstituted to trisubstituted by identical or different substituents from the series consisting of $C_1$-$C_{12}$-alkyl, F, Cl, Br, $CF_3$, $C_1$-$C_4$-alkoxy, $C_1$-$C_4$-alkoxy which is monosubstituted to hexasubstituted by identical or different substituents from the series consisting of F and Cl, $C_1$-$C_4$-alkoxy-$C_1$-$C_4$-alkyl, $C_1$-$C_4$-alkoxyethyleneoxy, $C_1$-$C_4$-alkylthio and $C_1$-$C_4$-alkylthio which is monosubstituted to hexasubstituted by identical or different substituents from the series consisting of F and

Cl;
$C_1$-$C_{12}$-alkyl-carbonyl,
$C_1$-$C_{12}$-alkoxy-carbonyl,
$C_5$-$C_7$-cycloalkyloxy-carbonyl which is optionally substituted by $C_1$-$C_6$-alkyl and/or $C_1$-$C_6$-alkoxy;
$C_5$-$C_7$-cycloalkyl-$C_1$-$C_6$-alkyloxy-carbonyl which is optionally substituted in the cycloalkyl moiety by $C_1$-$C_6$-alkyl and/or $C_1$-$C_6$-alkoxy; and
the group -CO-$NY^1Y^2$ in which
$Y^1$ and $Y^2$ are identical or different and have the following meanings:
hydrogen,
$C_1$-$C_6$-alkyl,
$C_5$-$C_7$-cycloalkyl which is optionally substituted by $C_1$-$C_6$-alkyl and/or $C_1$-$C_6$-alkoxy, and also
benzyl which is optionally substituted by the abovementioned benzyl substituents,

D represents hydrogen or methyl and

n represents 0, 1 or 2.

**3.** Process for the preparation of compounds of the formula (I) according to Claim 1, characterized in that

a) aminoalcohols of the formula (II)

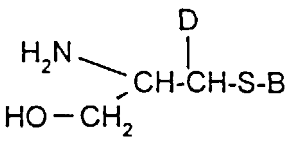

(II)

in which
B and D have the meaning given in Claim 1
are reacted with a carboxylic acid of the formula (III)

$$A\text{-}COOH \qquad (III)$$

in which

A has the meaning given in Claim 1

and with a dehydrating agent, if appropriate in the presence of a diluent;
or

b) amidoalcohols of the formula (IV)

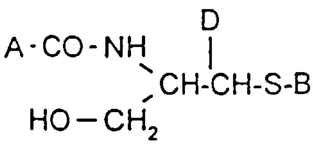

(IV)

in which
A, B and D have the abovementioned meanings
are reacted with a dehydrating agent, if appropriate in the presence of a diluent;
or

c) amide derivatives of the formula (V)

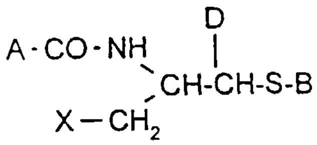

(V)

in which
A, B and D have the abovementioned meanings and

X represents a leaving group, such as halogen, alkylsulphonyloxy or arylsulphonyloxy,

are reacted with a base, if appropriate in the presence of a diluent;
or

d) oxazolines of the formula (VI)

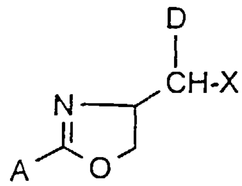

(VI)

in which
A, D and X have the abovementioned meanings are reacted with thiophenols of the formula (VII)

H-S-B (VII)

in which
B has the abovementioned meaning
in the presence of a base and if appropriate in the presence of a diluent;
and, if appropriate

e) the compounds of the formula (Ia)

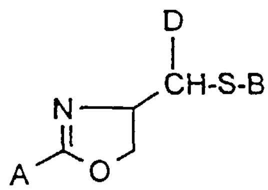

(Ia)

in which
A, B and D have the abovementioned meanings and
which can be obtained by processes (a) to (d)
are reacted with an oxidant, if appropriate in the presence of a diluent and if appropriate in the presence of a reaction auxiliary.

**4.** Pesticide which comprises at least one compound of the formula (I) according to Claim 1.

**5.** Method of combating animal pests, characterized in that compounds of the formula (I) according to Claim 1 are allowed to act on animal pests and/or their environment.

6. Use of compounds of the formula (I) according to Claim 1 for combating animal pests.

7. Process for the preparation of pesticides, characterized in that compounds of the formula (I) according to Claim 1 are mixed with extenders and/or surface-active agents.

**Revendications**

1. Composés de formule (I)

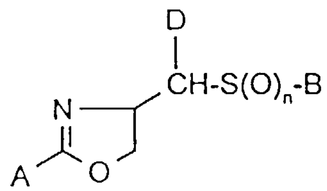

(I)

dans laquelle

A représente un groupe phényle qui est substitué une à cinq fois identiques ou différentes par un halogène, un groupe alkyle en $C_1$ à $C_6$, un groupe alkoxy en $C_1$ à $C_6$, un groupe alkylthio en $C_1$ à $C_6$, un groupe halogénalkyle en $C_1$ à $C_6$, un groupe halogénalkoxy en $C_1$ à $C_6$ ou halogénalkylthio en $C_1$ à $C_6$ ;

B est un groupe phényle qui est substitué, le cas échéant, une à quatre fois identiques ou différentes par un halogène, un groupe alkyle en $C_1$ à $C_{18}$, un groupe cyano, un groupe (alkoxy en $C_1$ à $C_8$)-(alkyle en $C_1$ à $C_8$),

un groupe halogénalkoxy en $C_1$ à $C_8$, un groupe halogénalkyle en $C_1$ à $C_4$, un groupe alkoxy en $C_1$ à $C_{18}$ qui est interrompu, le cas échéant par 1 à 3 autres atomes d'oxygène, un groupe alkylthio en $C_1$ à $C_{18}$, un groupe halogénalkylthio en $C_1$ à $C_8$, un groupe 3,4-difluorométhylènedioxo, un groupe 3,4-tétrafluoréthylènedioxo, un groupe benzylimino-oxyméthyle éventuellement substitué par un reste alkyle en $C_1$ à $C_4$, cycloalkyle en $C_3$ à $C_6$ et/ou halogéno, un groupe cyclohexyle ou un groupe cyclohexyloxy dont chacun est substitué le cas échéant par un reste alkyle en $C_1$ à $C_6$, alkoxy en $C_1$ à $C_6$, cyclohexyle ou phényle ; un groupe pyridyloxy éventuellement substitué une ou deux fois identiques ou différentes par un reste halogéno, alkyle en $C_1$ à $C_4$ ou halogénalkyle en $C_1$ à $C_4$ ; un groupe phényle, phénylcarbonyle, benzyle, phénoxy, phénylthio, benzyloxy, benzyloxycarbonyle ou benzylthio dont chacun porte, le cas échéant, un à trois substituants, identiques ou différents, alkyle en $C_1$ à $C_{12}$, halogéno, halogénalkyle en $C_1$ à $C_4$, alkoxy en $C_1$ à $C_6$, halogénalkoxy en $C_1$ à $C_6$, (alkoxy en $C_1$ à $C_6$)-(alkyle en $C_1$ à $C_6$), (alkoxy en $C_1$ à $C_6$)éthylène-oxy, alkylthio en $C_1$ à $C_6$ et/ou halogénalkylthio en $C_1$ à $C_6$ ; un groupe (alkyle en $C_1$ à $C_{12}$)-carbonyle, un groupe (alkoxy en $C_1$ à $C_{12}$)-carbonyle,

un groupe (cycloalkyloxy en $c_5$ à $C_7$)-carbonyle qui porte, le cas échéant, un substituant alkyle en $C_1$ à $C_6$ et/ou alkoxy en $C_1$ à $C_6$ ; un groupe (cycloalkyle en $C_5$ à $C_7$)-(alkyloxy en $C_1$ à $C_6$)-carbonyle qui est substitué le cas échéant dans la partie cycloalkyle par un reste alkyle en $C_1$ à $C_6$ et/ou alkoxy en $C_1$ à $C_6$ ; et le groupement $-CO-NY^1Y^2$ où $Y^1$ et $Y^2$ sont identiques ou différents et ont les définitions suivantes :
hydrogène,
alkyle en $C_1$ à $C_6$,

cycloalkyle en $C_5$ à $C_7$ éventuellement substitué par un reste alkyle en $C_1$ à $C_6$ et/ou alkoxy en $C_1$ à $C_6$, ainsi que
benzyle, qui est substitué le cas échéant par les substituants indiqués ci-dessus pour le groupe benzyle,

D est l'hydrogène ou un groupe méthyle et
n a la valeur 0,1 ou 2.

**2.** Composés de formule (I) suivant la revendication 1, dans laquelle

A est un groupe phényle qui est substitué une à cinq fois identiques ou différentes par
F, Cl, Br, un reste alkyle en $C_1$ à $C_3$, alkoxy en $C_1$ à $C_3$, alkylthio en $C_1$ à $C_3$, un reste alkyle en $C_1$ ou $C_2$ portant, le cas échéant, un à cinq substituants F et/ou Cl identiques ou différents, un groupe alkoxy en $C_1$ à $C_4$ substitué une à cinq fois identiques ou différentes par F et/ou Cl, un groupe $SCF_3$ ou $SCHF_2$ ;
B est un groupe phényle qui est substitué, le cas échéant, une à quatre fois identiques ou différentes par
F, Cl, Br,
alkyle en $C_1$ à $C_{18}$,
cyano,
(alkoxy en $C_1$ à $C_6$)-(alkyle en $C_1$ à $C_8$),
alkoxy en $C_1$ à $C_8$ substitué une à six fois identiques ou différentes par F et/ou Cl,
alkyle en $C_1$ ou $C_2$ substitué une à cinq fois identiques ou différentes par F et/ou Cl,
alkoxy en $C_1$ à $C_{18}$ et $-(OC_2H_4)_{1-3}$-O-(alkyle en $C_1$ à $C_6$),
alkylthio en $C_1$ à $C_{12}$,
alkylthio en $C_1$ à $C_8$ substitué une à six fois identiques ou différentes par F et/ou Cl,
3,4-difluorométhylènedioxo,
3,4-tétrafluoréthylènedioxo,
les groupements

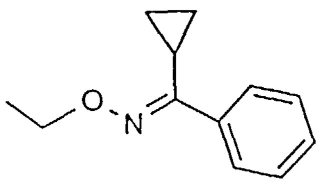
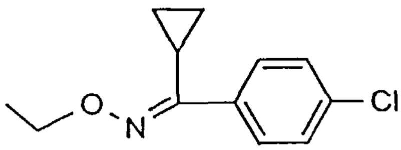
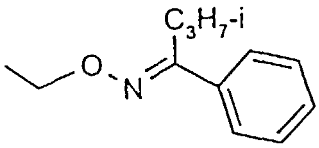
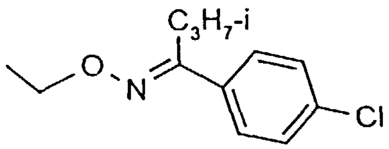
,

cyclohexyle ou cyclohexyloxy substitué chacun le cas échéant par un groupe alkyle en $C_1$ à $C_4$, alkoxy en $C_1$ à $C_4$, cyclohexyle ou phényle ;
pyridyloxy éventuellement substitué une ou deux fois identiques ou différentes par F, Cl ou $CF_3$ ;
phényle, phénylcarbonyle, benzyle, phénoxy, phénylthio, benzylthio, benzyloxy, benzyloxycarbonyle ou benzylthio dont chacun est substitué, le cas échéant, une à trois fois identiques ou différentes par
un reste alkyle en $C_1$ à $C_{12}$, F, Cl, Br, $CF_3$, alkoxy en $C_1$ à $C_4$, alkoxy en $C_1$ à $C_4$ portant un à six substituants F et/ou Cl identiques ou différents, (alkoxy en $C_1$ à $C_4$)-(alkyle en $C_1$ à $C_4$), (alkoxy en $C_1$ à $C_4$)éthylène-oxy, alkylthio en $C_1$ à $C_4$, alkylthio en $C_1$ à $C_4$ portant un à six substituants F et/ou Cl identiques ou différents ;
(alkyle en $C_1$ à $C_{12}$)-carbonyle,
(alkoxy en $C_1$ à $C_{12}$)-carbonyle,
(cycloalkyloxy en $C_5$ à $C_7$)-carbonyle qui est substitué, le cas échéant, par un à six restes alkyle en $C_1$ à $C_6$ et/ou alkoxy en $C_1$ à $C_6$ ;
(cycloalkyle en $C_5$ à $C_7$)-(alkyloxy en $C_1$ à $C_6$) qui est substitué le cas échéant dans la partie cycloalkyle par un reste alkyle en $C_1$ à $C_6$ et/ou alkoxy en $C_1$ à $C_6$ ; et
le groupement $-CO-NY^1Y^2$, dans lequel
$Y^1$ et $Y^2$ sont identiques ou différents et ont les définitions suivantes :
hydrogène,
alkyle en $C_1$ à $C_6$,

cycloalkyle en $C_5$ à $C_7$ qui est substitué le cas échéant par un reste alkyle en $C_1$ à $C_6$ et/ou alkoxy en $C_1$ à $C_6$, ainsi que benzyle, qui est substitué le cas échéant par les substituants du groupe benzyle mentionnés ci-dessus,

D est l'hydrogène ou le groupe méthyle et

n a la valeur 0,1 ou 2.

**3.** Procédé de production de composés de formule (I) suivant la revendication 1, caractérisé en ce que

a) on fait réagir des amino-alcools de formule (II)

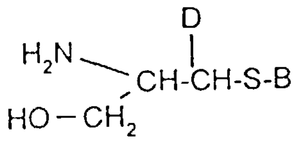

(II)

dans laquelle

B et D ont la définition indiquée dans la revendication 1,

avec un acide carboxylique de formule (III)

A-COOH (III)

dans laquelle

A a la définition indiquée dans la revendication 1,

avec un agent déshydratant, le cas échéant en présence d'un diluant ;
ou bien
b) on fait réagir des amido-alcools de formule (IV)

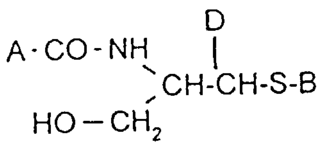

(IV)

dans laquelle

A, B et D ont la définition indiquée ci-dessus, avec un agent déshydratant, le cas échéant en présence d'un diluant ;

ou bien
c) on fait réagir des dérivés d'amides de formule (V)

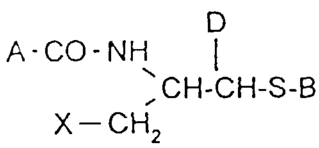

(V)

dans laquelle

A, B et D ont la définition indiquée ci-dessus ; et
X représente un groupe partant tel qu'un halogène, un groupe alkylsulfonyloxy ou arylsulfonyloxy,

avec une base, le cas échéant en présence d'un diluant ; ou bien
d) on fait réagir des oxazolines de formule (VI)

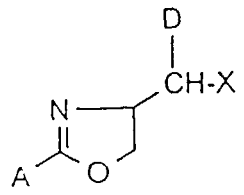

(VI)

dans laquelle

A, D et X ont la définition indiquée ci-dessus,

avec des thiophénols de formule (VII)

H-S-B (VII)

dans laquelle

B a la définition indiquée ci-dessus en présence d'une base et, éventuellement, en présence d'un diluant ;

et, le cas échéant
e) on fait réagir les composés obtenus selon les procédés (a) à (d), de formule (Ia)

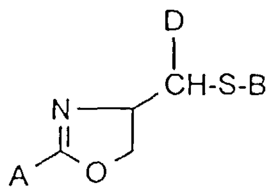

(Ia)

dans laquelle

A, B et D ont la définition indiquée ci-dessus,

avec un agent oxydant, le cas échéant en présence d'un diluant et en la présence éventuellement d'une substance auxiliaire de réaction.

**4.** Compositions pesticides, caractérisées par une teneur en au moins un composé de formule (I) suivant la revendication 1.

**5.** Procédé pour combattre des parasites animaux, caractérisé en ce qu'on fait agir des composés de formule (I) suivant la revendication 1 sur des parasites animaux et/ou sur leur milieu.

**6.** Utilisation de composés de formule (I) suivant la revendication 1 pour combattre des parasites animaux.

**7.** Procédé de préparation de compositions pesticides, caractérisé en ce qu'on mélange des composés de formule (I) suivant la revendication 1 avec des diluants et/ou des agents tensio-actifs.